# EUROPEAN PATENT APPLICATION

(11) **EP 1 471 153 A2**
(43) Date of publication of application: **27.10.2004**
(21) Application number: 04006035.2
(22) Date of filing: 13.03.2004
(51) Int. Cl.: C12Q 1/68

(54) **Transcriptional activity assay**

(30) Priority: 21.03.2003 EP 03006263
(71) Applicant: F. HOFFMANN-LA ROCHE AG, 4070 Basel (CH)
(72) Inventor: Certa, Ulrich, 4123 Allschwil (CH); Foser, Stefan, 79639 Grenzach-Wyhlen (DE); Weyer, Karl, 79415 Bad Bellingen (DE)

(57) **Abstract**

The present invention provides a method for determining the biological activity of a compound comprising the steps of a) contacting a host with a compound; b) determining the general transcriptional gene response of the host; and c) quantitating the general transcriptional gene response induced by said compound.

## Description

The present invention relates to a novel method for determining the biological activity of compounds which can modulate gene transcription, and based on this method, a method for the determination of the relative biological activity of a compound and a method for the identification of compounds which modulate gene transcription. The methods of this invention are particularly useful for identifying compounds that may be of use in the treatment of diseases which depend on the modulation of gene transcription within affected cells and tissues.

The biological activity of compounds that regulate cellular functions such as cell proliferation or viral protection is commonly determined by measuring increases or decreases of binding activities of these factors and/or compounds either to native or recombinant receptors in vitro, or in cell-based binding assays. A disadvantage of these assays for the evaluation of the biological activity of compounds resides in the fact that the binding affinity does not necessarily correlate with the biological activity of a compound.

A more direct and, therefore, more reliable determination of the biological activity of compounds can be achieved by well known assays such a cell proliferation, cell survival or cell migration assays. Such assays are, however, more cumbersome, especially for larger scale screenings. In many cases, compounds which regulate such cellular activities also modulate gene transcription. Thus, the biological activity of such compounds may be determined by measuring the modulation of gene transcription, which is commonly done by reporter assays, which necessitate the generation of recombinant cells expressing a suitable reporter construct. Alternatively, the biological activity of such compounds may be determined by measuring the expression of suitable target genes, eg. by Northern Blot analysis or quantitative PCR, which is, however, not efficient.

The problem of the present invention was to provide a reliable and easy method for the determination of the biological activity of a modulator of transcriptional activity which lacks the disadvantages of binding assays, but is more efficient than the commonly used activity assays.

Surprisingly, it was found that the biological activity of a compound can be determined more reliably by using a novel method based on the determination of the general transcriptional gene response induced by said compound comprising quantitation of signal intensities of genes which are transcriptionally regulated by said compound.

The present invention provides a novel method for determining the biological activity of a compound which can modulate gene transcription, comprising the steps of a) contacting a host with a compound; b) determining the general transcriptional gene response of the host; and c) quantitating the general transcriptional gene response induced by said compound. Preferably, the biological activity of said compound is given by the sum of the normalized average difference of signal intensities quantitated in step c). In a more preferred embodiment of the present invention the general transcriptional gene response is determined by an DNA array technology. In an even more preferred embodiment, the array technology is an oligonucleotide array technology. Preferably the DNA or oligonucleotide arrays are high density DNA or oligonucleotide arrays. Such arrays, and methods of analyzing DNA or RNA using such arrays have been described previously, eg. in EP0834575, EP0834576, WO96/31622, US5837832 or WO98/30883. WO97/10365 provides methods for monitoring of expression levels of a multiplicity of genes using high density oligonucleotide arrays. In a most preferred embodiment, the oligonucleotide arrays are GeneChip arrays.

The term "general transcriptional gene response "as used herein refers to the biological activity of a compound given by the sum of the normalized average difference of signal intensities of genes regulated by said compound in a responsive host. The determination of the general transcriptional gene response is achieved by hybridizing labeled cDNA from the treated hosts with DNA or more preferably oligonucleotide arrays. The raw data of the arrays is then measured, eg. with a confocal laser scanner (Hewlett Packard, U.S.) as a non-limiting example, and pixel levels are analyzed using commonly known methods. The expression level for each gene is calculated as a normalized average difference of intensity as compared to hybridization to mismatched oligonucleotides, and expressed in arbitrary units (AD). Four criteria are applied to select differentially expressed genes: (1) The intensity value of treated cells was at least two fold higher than the intensity value of untreated cells. (2) The intensity value in the treated cells should be higher or at least 50 arbitrary units (AD). (3) The intensity value of the untreated cells should be lower than 50 AD. (4) The standard deviation has to be significantly lower than the absolute change in average difference and the calculated confidence level of a gene is set to greater than 97% (p value < 0.03). The general transcriptional gene response of the host induced by a compound is then quantitated by obtaining the sum of the normalized average difference of signal intensities (sum of arbitrary units, ∑AD).

The non-limiting examples described hereinafter demonstrate that the results obtained with this novel method more reliably correlate with biological activity than do binding assays, with the additional advantage of being efficient. They also show that determining the general transcriptional gene response by the method of the present invention is more reliable than the determination of the transcriptional expression levels of an individual gene or a small selection of genes.

The host used for the method of the present invention may be a whole non-human organism, which includes, but is not limited to a transgenic or a knockout animal; specific types of tissue from any species, which includes, but is not limited to liver, heart, kidney, or primary cell isolates, such as blood cells or primary cells isolated from a tissue, or cell lines. The host or hosts used to determine the biological activity of a compound by the present invention has to be responsive to a compound of interest. The term "responsive" as used herein refers to the ability of a host to exhibit elevated or reduced transcription of genes in response to a compound. As a non-limiting example, a melanoma cell line ME15 (CNCM I-2546 in WO02/18633) can be used to determine the biological activity of PEG-IFN or PEG-IFN isoforms by the method of the present invention.

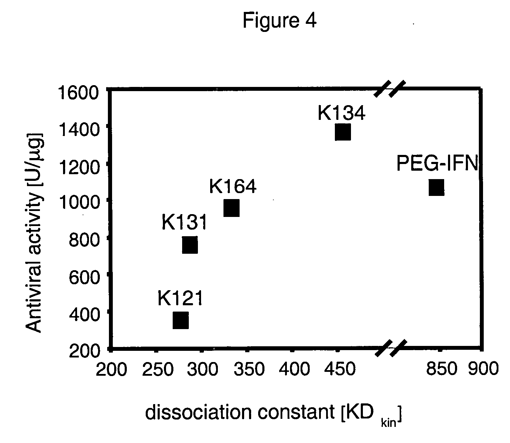

The major advantages of the present invention, as compared to the commonly used activity assays or methods for determining gene transcription such as reporter assays or Northern Blotting are that the method does not require the preparation of any cell lines transfected with reporter constructs, and it allows for an easy and efficient analysis of a multitude of genes that are transcriptionally regulated, thus rendering the activity assays more efficient and reliable. Furthermore, as shown in Figure 4, binding activities do not always correlate with biological activity, while the determination of the transcriptional activity of compounds based on the determination of the general transcriptional gene response does correlate. Thus, the method of the present invention allows a more accurate determination of the biological activity of compounds than do binding assays.

In one embodiment, the compound of the method hereinbefore described is a modified compound, more preferably a modified protein, even more preferably a pegylated protein, or most preferably, a specific isolated isoform of a pegylated protein. Preferably, the pegylated protein is erythropoietin (EPO) or interferon (IFN).

The term "PEG-IFN" as used herein includes IFN-αs derived from any natural material (e.g., leukocytes, fibroblasts, lymphocytes) or material derived therefrom (e.g. cell lines), or those prepared with recombinant DNA technology. Details of the cloning of IFNα and the direct expression thereof, especially in E. coli, have been the subject of many publications. The preparation of recombinant IFNαs is known, for example from Goeddel et al. (1980) *Nature* 284, 316-320 and (1981), *Nature* 290, 20-26, and European Patents Nos. 32134, 43980 and 211148. There are many types of IFNα such as IFNαI, IFNα2; and further their subtypes including but not limited to IFNα2A, IFNα2B, IFNα2C and IFNαII (also designated IFNαII or ω-IFN). The term "IFNα" also includes consensus IFNα available from Amgen or mixtures of natural and/or recombinant IFNαs. The use of IFNα2A is preferred. The manufacture of IFNα2A is described in European Patents Nos. 43980 and 211148.

The IFN-α is conjugated to a polymer such as a polyalkylene glycol (substituted or unsubstituted), for example, polyethylene glycol, to form PEG-IFN-α conjugate. Conjugation may be accomplished by means of various linkers known in the art, in particular by linkers such as those disclosed in European Patent Applications, Publication Nos. 0510356, 0593868 and 0809996. The molecular weight of the polymer, which is preferably polyethylene glycol, may range from 300 to 70.000 daltons, and one or more, preferably one to three, polymers may be conjugated to the IFN-α. A preferred PEG-IFN-α conjugate has the formula: 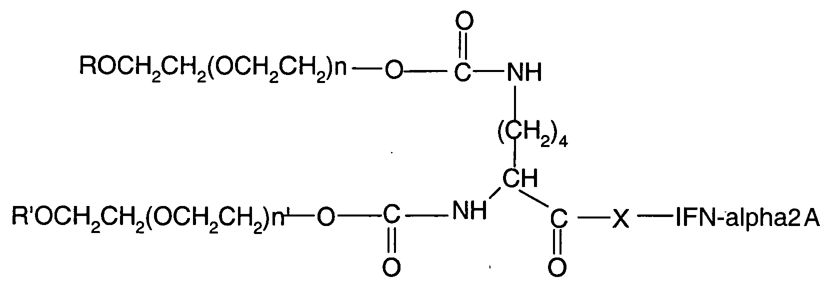 where R and R' are methyl, X is NH, and n and n' are individually or both either 420 or 520.

In another preferred embodiment of the method hereinbefore described, the transcriptionally regulated genes are one or more genes selected from the group consisting of Seq ID No. 1 to 29, which are also listed in Table 1.

The method of the present invention is suitable for the analysis of any compound that modulates gene transcription in any type of host. Modulation of gene transcription includes both upregulation or downregulation of gene transcription.

The method hereinbefore described is the basis for a method for determining the relative biological activity of a modified compound, comprising measuring the biological activity of the modified compound and the respective unmodified compound by the method hereinbefore described, wherein the relative transcriptional activity of the modified protein is determined by calculating the ratio of the biological activity of the modified compound and the biological activity of the unmodified compound. Preferably, the modified compound is a chemically or enzymatically modifed compound. More preferably, the modified compound is a pegylated protein. Thus, this method provides an easy assay to compare the biological activity of modified compounds with their unmodified counterparts. In a preferred embodiment, the present invention allows to select the isoform of a compound with the highest biological activity isolated from a mixture of isoforms, most preferably from pegylated proteins.

The compounds whose biological activity can be determined by the method of this invention include any compound that can modulate gene transcription. These compounds can be proteins, polypeptides, nucleic acids, polymers or small molecules. Many proteins, polypeptides, nucleic acids, polymers or small molecules can modulate gene transcription, either directly, by acting as transcription factors, or indirectly, by inducing signaling cascades that ultimately up- or down-regulate gene transcription or alternatively, by interfering with other compounds that modulate gene transcription. The following list of compounds whose biological activity can be determined by the method of the present invention exemplifies in a non-limiting way the kinds of compounds, including proteins and polypeptides, for which the method of the present invention can be used.

Among these non-limiting examples are hormones, such as retinoid acic, that induce nuclear hormone receptors, which are transcription factors (A. Rowe: Retinoid X receptors, *Int. J. Biochem. Cell Biol.,* 29 (1997), 275-278; and CK. Glass: Some new twists in the regulation of gene expression by thyroid hormone and retinoic acid receptors, *J. Endorinol,* 150 (1996), 349-357). The method of the present invention may be used to determine the biological activity of such hormones and their nuclear hormone receptors, and it may also be used to identify compounds that interfere with the transcriptional activity of such nuclear hormone receptors. Thus, the present invention may, for example, be useful for identifying and analyzing inhibitors of nuclear hormone receptors such as RXR or LXR.

Further to hormones that activate nuclear hormone receptors, the method of this invention may also be used to determine the biological activity of other hormones which activate signaling cascades that lead up to the modulation of gene transcription. As a non-limiting example, such hormones include erythropoietin (Epo), which is known to modulate gene transcription (Z.Z.Chong et al: Hematopoietic factor erythropoietin foster neuroprotection through novel signal transduction cascades, *J. Cereb. Blood Flow Metab.,* 22 (2002), 503-514; D.M. Wojchowski and T.C. He: Signal transduction in the erythropoietin receptor system, *Stem. Cells,* 11 (1993), 381-392; J.L. Spivak: The mechanism of action of erythropoietin, *Int. J. Cell Cloning,* 4 (1986), 139-166; and L. Mulcahy: The erythropoietin receptor, *Semin. Oncol.* 28 (2001), 19-23).

Cytokines and growth factors are known to induce signaling cascades that lead to a modulation of gene transcription. Thus, as a non-limiting example, the method of the present invention may also be used to identify agonists or antagonists of cytokines and growth factors, and to determine their biological activity. Such cytokines and growth factors include, but are not limited to, Interferons, Tumor Necrosis Factor, Vascular Endothelial Growth Factor (T. Hanada and A. Yoshimura: Regulation of cytokine signaling and inflammation, *Cytokine Growth Factor Rev.,* 13 (2002), 413-421; and Y. Sato et al.: Properties of two VEGF receptors, Flt-1 and KDR, in signal transduction, *Ann. NY. Acad. Sci.,* 902 (2000), 201-207).

Further to this, the method of the present invention may be used to determine the biological activity of modified or mutated proteins or polypeptides. Modification may be achieved either chemically or enzymatically. A non-limiting example for this application is the determination of the biological activity of different isoforms of pegylated Interferon, which is shown in the Figures attached to this application. Thus, the present invention provides for determining the relative biological activity of a modified compound, comprising measuring the biological activity of the modified compound and the respective unmodified compound by the method hereinbefore described, wherein the relative transcriptional activity of the modified protein is determined by calculating the ratio of the biological activity of the modified compound and the biological activity of the unmodified compound. Preferably, the modified compound is a chemically or enzymatically modifed compound. Most preferably, the modified compound is a pegylated protein.

The method of the present invention may also be used for identifying and characterizing modulators of enzymes which, as a downstream event, can cause a modulation of gene transcription. A non-limiting example for such enzymes are phosphodiesterases (PDE), preferably PDE4, most preferably PDE4D isoenzymes. The PDE4D isoenzymes are preferably selected from the group consisting of PDE4D1, PDE4D2, PDE4D3, PDE4D4, PDE4D5, PDE4D6, PDE4D7 and PDE4D8. PDEs degrade cyclic AMP, a second messenger which can modulate gene transcription (G.S. McKnight: Cyclic AMP second messenger systems, *Curr. Opin.Cell Biol.,* 3 (1991) 213-7), and potent PDE inhibitors were found to modulate gene transcription (B. Wagner et al.: 7-Benzylamino-6chloro-2piperazino-4-pyrrolidino-pteridine, a potent inhibitor of cAMP-specific phosphodiesterase, enhancing nuclear protein binding to the CRE consensus sequence in human tumour cells, *Biochemcial Pharmacology,* 63 (2002) 659-668; Y. Nishio et al.: Cilostazol, a cAMP phosphodiesterase inhibitor, attenuates the production of monocyte chemoattractant protein-1 in response to tumor necrosis factor-alpha in vascular endothelial cells, *Hormone* 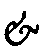 *Metabolic Research,* 29 (1997) 491-495). Thus, the method of the present invention may be used for screening of modulators of such enzymes.

Among the compounds that can be identified and analyzed are also functional antibodies. Functional antibodies are antibodies that either inhibit or stimulate the function(s) of the protein that they bind to. This type of antibodies has been raised against numerous target proteins, and some of these antibodies are also of therapeutic value. Amongst these are, as a non-limiting example, anti-Her2/ErbB2 antibodies, also called Herceptin antibodies. These antibodies are being successfully used in the treatment of breast cancer. They function by inhibiting the activation of human epidermal growth factor receptor Her2/ErbB2, which plays a pivotal role in many types of cancer, including breast cancer. Like many other growth factor receptors, Her2, when bound by its ligand(s), mediates signaling including modulation of gene transcription. It has been shown that Herceptin is capable of modulating gene transcription in cells that express Her2/ErbB2 (A.H. Talukder et al.: Antihuman epidermal growth factor receptor 2 antibody herceptin inhibits autocrine motility factor (AMF) expression and potentiates antitumor effects of AMF inhibitors, *Clin. Cancer Res.* 8 (2002), 3285-3289; and R. Mandler et al.: Modification in synthesis strategy improve the yield and efficacy of geldanamycin-herceptin immunoconjugates, *Bioconjugate Chemistry,* 13 (2002), 786-791). Currently, eight different Herceptin antibodies exist, and more may still be produced. Generally, in the case of functional antibodies, the method of the present invention may be used in determining which antibody displays the highest biological activity and would be the most suited for therapeutic use. Thus, the method of the present invention is also suitable for determining the biological activity of antibodies, eg. for the identification of inhibitory or stimulating antibodies. In a preferred embodiment, the antibody is an antibody of therapeutic value. In a more preferred embodiment, the antibody is a Herceptin antibody.

For the identification and analysis of a compound that acts indirectly by inhibiting or stimulating the biological activity of a second compound, such as a hormone, cytokine, growth factor, enzyme etc., the present invention provides a method for identifying compounds that indirectly modulate gene transcription comprising determining the biological activity of a first compound which is a known modulator of gene transcription by the methods hereinbefore described in the presence or absence of a second compound, wherein a compound which indirectly modulates gene transcription is identified if the biological activity of the first compound measured in the presence of said second compound is significantly different from the biological activity of the first compound measured in the absence of said second compound.

The present invention provides a compound identified by the methods hereinbefore described which modulates gene transcription. A use of the methods hereinbefore described for identifying a compound which modulates gene transcription is provided as well. The present invention also provides a kit comprising components for carrying out the methods hereinbefore described. Preferably, said components comprise a DNA array, more preferably an oligonucleotide array.

Furthermore, a pharmaceutical composition comprising a compound identified by the methods hereinbefore described and a pharmaceutically acceptable carrier is provided. Any conventional carrier material can be utilized. The carrier material can be an organic or inorganic one suitable for eteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, stabilizers, emulsifying agents, buffers and the like may be added in accordance with accepted practices of pharmaceutical compounding.

Additionally, the present invention provides a method for the production of a pharmaceutical composition comprising the steps of identifying a compound by the method of the present invention, modifying the identified compound and formulating the compound obtained with a pharmaceutically acceptable carrier or diluent.

The present invention also provides a use of a compound identified by the methods hereinbefore described or a pharmaceutically acceptable salt thereof for the preparation of a medicament for the treatment of a disease, preferably of a viral disease or of a cancer.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the identified agents wherein the parent agent is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, benzenesulfonic, toluenesulfonic, methanesulfonic, ethane disulfonic, oxalic, isethionic, and the like.

Also claimed are the methods, compounds, kits and uses as hereinbefore described, especially with reference to the foregoing examples.

Having now generally described this invention, the same will become better understood by reference to the specific examples, which are included herein for purpose of illustration only and are not intended to be limiting unless otherwise specified, in connection with the following figures.

### Brief description of the figures:

**Figure 1:**
   Analytical IEC-HPLC of 180µg of PEG-IFN. An analytical strong-cation exchange column was used to separate the positional isomers (TOSOH-BIOSEP, SP-5PW, 10 µm particle size, 7.5 mm diameter and 7.5 cm length).
**Figure 2:**
   (A) Specific antiviral activity of the 9 positional isomers and PEG-IFN
      The figure shows the specific antiviral activity [U/µg] of the positional isomers. The antiviral activity was determined in MDBK cells infected with vesicular stomatitis virus.
   (B) Specific anti-proliferative activity of the isomers and PEG-IFN
      This figure indicates the specific inhibition of proliferation [OD₄₉₀ₙₘ/µg] on the ME15 cells with 100 U/ml of the each positional isomer and PEG-IFN.
      The results present the averages of three assays performed independently. The specific antiviral activity and specific anti-proliferative activity of PEG-IFN is given for comparison (hatched line).
**Figure 3:**
   Time dependent inhibition of proliferation in a melanoma cell line (ME15). The ME15 cell line was cultured in presence of different concentrations (500, 125, and 15 U/ml) IFN, PEG-IFN and isomers over 5 days. The cell proliferation was measured every 24h. The control cell density after 5 days was set as 100% as references. The data are derived from three independent culture replicates.
**Figure 4:**
   Correlation of the kinetic dissociation constant and specific antiviral activity of the PEG-IFN and isomers with high (K134), intermediate (K131, K164) and low (K121) specific antiviral activity.
**Figure 5:**
   (A) Correlation of specific antiviral activity, specific anti-proliferative activity and specific transcriptional response of each isomer. The PEG-IFN was set as 100% value reference. The hatched line indicates the biological and transcriptional activity of PEG-IFN. The sum ∑ relative mRNA abundance (AD) is illustrated as the sum of all inducible genes for each isomer.
   (B) Hierarchical clustering of AD of the different general transcriptional gene response from 100 U/ml IFN, PEG-IFN and the isomers. The incubation time of the IFN and the pegylated variants was 48h; the cell line used is an IFN-sensitive melanoma cell line (ME15). The conditions for the data analysis are described in the text. The data of the different expression profiles of the genes are reported in Table 3.
**Figure 6:**
   3D-Structure based Model for PEG-IFN mediated interferon responses. Arrangement of the positional PEG-IFN isomers in four groups according to the specific antiviral and anti-proliferation activity. The high resolution structure of human interferon alpha-2a was determined with NMR spectroscopy (Klaus et al.: The three-dimensional high resolution structure of human interferon alpha-2a determined by heteronuclear NMR spectroscopy in solution. J. *Mol. Biol.* **274** (1997); 661-675). The pegylation sites of interferon alpha-2a are colored red.

### Examples

### Antiviral and anti-proliferative roles of IFN

Interferon alpha-2a (IFN) plays an important role in mediating antiviral and anti-proliferative responses and in modulating immune responses (G.R. Stark: How cells respond to interferons, *Annu. Rev. Biochem.* **67** (1998), 227-264). These biological activities make IFN important as an inhibitor of disease pathogenesis and useful in the treatment of viral infection and some cancers (S. Zeuzem, et al. : Peginterferon alfa-2a in patients with chronic hepatitis C, *New England Journal of Medicine* **343** (2000), 1666-1172). Since 1986 recombinant human IFN (ROFERON-A) has become an important therapeutic agent to treat patients with viral and oncological diseases (S. Zeuzem et al.: Peginterferon alfa-2a (40 kDa) monotherapy: a novel agent for chronic hepatitis C therapy, *Expert Opinion on Investigational Drugs* **10** (2001), 2201-2213).

The success of IFN treatment in the clinic is partly limited due to its short serum half-life (4-6 h). Peak concentrations of IFN occur 3-8 h following iv. or sc. administration and large fluctuations in serum concentrations can occur after each dose. After 24 h , the amount of exogenous IFN remaining in the body is negligible such that the thrice-weekly regimen used is unlikely to maintain concentrations required for antiviral activity (J.M. Harris et al.: Pegylation: A novel process for modifying pharmacokinetics, *Clin. Pharmacokinet.* **40** (2001), 539-551; P. Bailonet al.: Rational design of a potent, long-lasting form of interferon: A 40 kDa branched polyethylene glycol-conjugated Interferon-2a for the treatment of hepatitis C *Bioconjugate Chem.* **12** (2001), 195-202).

Administration frequency of IFN is dependent on the disease. For example, the standard therapeutic application of IFN consists of 3 million international units administered subcutaneously three times per week for up to 48 weeks (J.M. Harris et al.: Pegylation: A novel process for modifying pharmacokinetics, *Clin. Pharmacokinet.* **40** (2001), 539-551), while certain oncological indications may require daily administration (P. Bailon et al.: Rational design of a potent, long-lasting form of interferon: A 40 kDa branched polyethylene glycol-conjugated Interferon-2a for the treatment of hepatitis C, *Bioconjugate Chem.* **12** (2001), 195-202). In order to improve the pharmacokinetic and pharmacodynamic properties of IFN, it was conjugated with poly(ethylene glycol) (PEG).

Since several years, studies have been carried out to assess the influence of the various PEG sizes and PEG types on in vitro and in vivo bioactivity. These results showed the important correlation of the PEG characteristics and the biological activity. For example, branched PEG moieties may more effectively cloak and protect IFN from proteolytic degradation than the linear PEG attachments and may also enhance the absorption and distribution properties of protein. Another effect is that increasing the molecular weight of the PEG moiety, decreases the clearance of the therapeutic molecule, thereby producing sustained exposure to the virus (J.M. Harris et al.: Pegylation: A novel process for modifying pharmacokinetics, *Clin. Pharmacokinet.* **40** (2001), 539-551). Based on this knowledge coupled with the latest advancements in pegylation procedures, a long-lasting, branched monopegylated IFN was developed (P. Bailon et al.: Rational design of a potent, long-lasting form of interferon: A 40 kDa branched polyethylene glycol-conjugated Interferon-2a for the treatment of hepatitis C, *Bioconjugate Chem.* **12** (2001), 195-202).

An interferon alpha-2a conjugated via an amide linkage with a branched 40kDa PEG (PEG-IFN), exhibits sustained adsorption and reduced renal clearance, resulting in strong antiviral pressure throughout a once-weekly dosing schedule (M.C. Perry and B. Jarvis: Peginterferon-α-2a (40 kD): A review of its use in the management of chronic hepatitis C, *Drugs* **15** (2001), 2263-2288; and M.W. Lamb and E.N. Martin: Weight-Based versus fixed dosing of peginterferon (40 kDa) alfa-2a, *The Annals of Pharmacotherapy* **36** (2002), 933-938). The characterization of this PEG-IFN mixture indicated 9 positional isomers which are monopegylated on lysines: K₃₁, K₄₉, K₇₀, K₈₀, K₁₁₂, K₁₂₁, K₁₃₁, K₁₃₄ and K₁₆₄. All positional isomers induced viral protection of MDBK cells in the Anti-Viral Assay.

### Example 1: Preparation of PEG-IFN

The PEG-IFN was manufactured by Hoffmann-La Roche Inc. The pegylated interferon alpha-2a was prepared by the conjugation of lysine ε-amino groups at the surface of the interferon molecule with an activated branched polyethylene glycol derivative of molecular weight 40000 Da (Bailon, P. et al.: Rational design of a potent, long-lasting form of interferon: A 40 kDa branched polyethylene glycol-conjugated Interferon-2a for the treatment of hepatitis C. *Bioconjugate Chem.* **12** (2001); 195-202; EP809996). The isolation and characterization of the isomers of PEG-IFN is described below. All other reagents and chemicals were of the highest purity available.

PEG-IFN consists of a mixture of monopegylated IFN molecules. In each molecule one single lysine is chemically modified by the attachment of a PEG moiety resulting in a mixture of several species. Due to the presence of eleven lysines and a free N-terminal amino group twelve monopegylated species are expected, but only nine lysine-modified species could be found (K31, K49, K70, K83, K112, K121, K131, K134 and K164).

### Example 2: Purification and characterization of positional PEG-IFN isomers

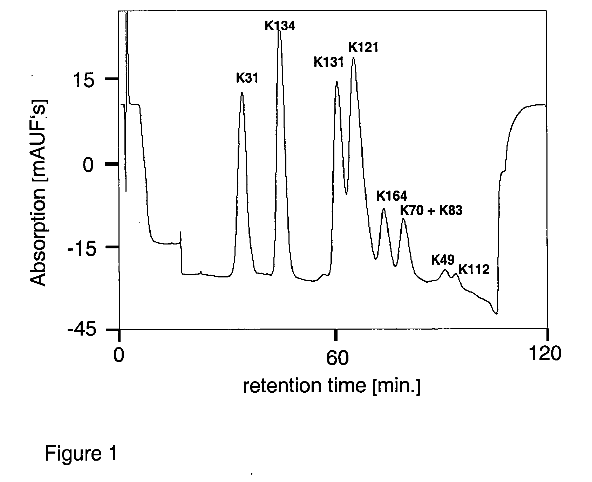

We developed a preparative two step chromatography method allowing efficient separation of these species. The purity of the prepared positional isomers could be checked by an analytical ion exchange method, see an example for an elution profile in Figure 1. Separation is based on local charge differences of the isomers resulting from the different pegylation sites on the protein moiety. The decrease of the baseline absorption towards the end of the chromatogram suggests that there were no other monopegylated species of IFN eluting at higher retention time. By two step separation nine positional isomers were purified, the characterization of the isomers indicate the pegylation sides are all on lysines: K31, K49, K70, K83, K112, K121, K131, K134 and K164.

### Example 3: Separation of the positional isomers

A two-step isolation and purification scheme was used to prepare the monopegylated isoforms of PEG-interferon alpha 2a.
a) The first step was a separation of the positional isomers on a preparative low pressure liquid chromatography column with a weak-cation exchange matrix (TOSOH-BIOSEP, Toyopearl CM-650S, Resin Batch no. 82A having a ion exchange capacity of 123 mEq/ml, the diameter of the column being 16 mm, the length 120 cm). A linear pH-gradient of increasing sodium acetate concentration (25 mM, pH 4.0 up 75 mM to pH 7.8) was applied at a flow rate of 0.7 mL/min. Detection was at 280 nm. With this chromatographic step species 1, 2, 5, 6 and a mixture of 3, 4, 4a, 7 and 8 could be collected, see Table 1.
b) The fractions were further separated and purified in the second preparation step. A preparative column with the same matrix as the analytical strong-cation exchange column as described above but larger dimensions (30 mm i.d. and 70 mm length), further a higher flow rate and an extended run time was used. As for the analytical method the column was pre-equilibrated with 3.4 mM sodium acetate, 10% ethanol and 1% diethylene glycol, adjusted to pH 4.4 (buffer A). After loading the PEG-IFN samples, the column was washed with buffer A, followed by an ascending linear gradient to 10 mM dibasic potassium phosphate, 10% ethanol and 1% diethylene glycol, adjusted to pH 6.6 (buffer B). The flow rate was 1.0 mL/min and the detection at 218 nm.

The protein concentration of the PEG-IFN alpha 2a isomer was determined by spectrophotometry, based on the 280 nm absorption of the protein moiety of the PEG-IFN alpha 2a.

An analytical elution profile of 180 µg of PEG-IFN alpha 2a is shown in Fig. 1. The result of this method is a separation into 8 peaks, 2 peaks with baseline separation and 6 with partial separation. The decrease of the baseline absorption towards the end of the chromatogram suggests that there were no other monopegylated species of IFN alpha 2a eluting at higher retention time.

In addition, looking carefully at the IEC-chromatogram a further peak close to the detection limit is visible between peaks 2 and 3 indicating the presence of additional positional isomers that should also contribute to the specific activity of the PEG-IFN alpha 2a mixture. Additional species were expected as the interferon alpha-2a molecule exhibits 12 sites for pegylation (11 lysines and the N-terminus). However, given the low abundance of the these species, they were not isolated and characterized.

Isomer samples derived from IEC optimization runs were investigated directly after the isolation (t = 0) and after 2 weeks of storage at 5°C. No significant differences were observed for the protein derived from IEC-peaks with regard to the protein content as determined by spectrometric methods; nor were any changes to be detected in the monopegylation site, the content of oligo-PEG-IFN alpha 2a, the amount of aggregates and the bioassay activity. Taking into account the relative abundance of the individual isomers - as determined by the IEC method - as well as the specific activities - as determined in the anti-viral assay - almost the total specific bioactivity of the PEG-IFN alpha 2a mixture used for their isolation is recovered (approximately 93%).

The analytical IE-HPLC was used to check the purity of the individual isomers with respect to contamination with other positional isomers in the IEC fractions. The peaks 2, 3, 4, 4a, 5 and 7 had more than 98%, the peaks 1 and 8 had 93% and peak 6 had 88 % purity.

**Table 1:**

| | | | |
|---|---|---|---|
| PEG-peptides identified by comparison of the Lys-C digest spectra of the isomers and the reference standard. | | | |

| Identified PEG Sites in the separated PEG-IFN Species | | | |
|---|---|---|---|
| Peak | | missing peaks in peptide map | |
| PEG-IFN | PEG site | Mᵣ (DA) | Sequence |
| Peak 1 | K³¹ | A, E | 24-49 |
| Peak 2 | K¹³⁴ | I, I' | 134-164 |
| Peak 3 | K¹³¹ | C | 122-131 ^{a} |
| Peak 4 | K¹²¹ | B, C | 113-131 |
| Peak 4a | K¹⁶⁴ | ^{b} | 134-164 ^{a,b} |
| Peak 5 | K⁷⁰ | D, F | 50-83 |
| Peak 6 | K⁸³ | D, H | 71-112 |
| Peak 7 | K⁴⁹ | E, F | 32-70 |
| Peak 8 | K¹¹² | B, H | 84-121 |

| | | | |
|---|---|---|---|
| ^{a} 132-133 too small to detect. | | | |
| ^{a,b} RP-HPLC. | | | |

The fractions were characterized by the methods described herein.

### Example 4: Analysis of the fractions by mass spectrometry peptide mapping

Mass spectra were recorded on a MALDI-TOF MS instrument (PerSeptive Biosystems Voyager-DE STR with delayed extraction). Each IEC fraction (Ion Exchange Chromatography) was desalted by dialysis, reduced with 0.02 M 1,4-dithio-DL-threitol (DTT) and alkylated with 0.2 M 4-vinyl pyridine. Then the proteins were digested with endoproteinase Lys-C (Wako Biochemicals) in 0.25 M Tris (tris(hydroxymethyl)-aminoethane) at pH 8.5 with an approximate enzyme to protein ratio of 1:30. The reaction was carried out over night at 37 °C.

A solution of 20 mg/ml α-cyano-4-hydroxycinnamic acid and 12 mg/ml nitrocellulose in acetone/isopropanol 40/60 (v/v) was used as matrix (thick-layer application). First, 0.5 µL of matrix was placed on the target and allowed to dry. Then, 1.0 µL of sample was added. The spectra were obtained in linear positive ionization mode with an accelerating voltage of 20.000 V and a grid voltage of 95 %. At least 190 laser shots covering the complete spot were accumulated for each spectrum. Des-Arg¹-bradykinin and bovine insulin were used for internal calibration.

### Example 5: High-performance liquid chromatography (RP-HPLC) Peptide Mapping

The peptides were characterized by reverse-phase high-performance liquid chromatography (RP-HPLC) Peptide Mapping. The IEC fractions were reduced, alkylated and digested with endoproteinase Lys-C as described for the MALDI-TOF MS peptide mapping. The analysis of the digested isomers was carried out on a Waters Alliance HPLC system with a Vydac RP-C18 analytical column (5 µm, 2.1 × 250 mm) and a precolumn with the same packing material. Elution was performed with an acetonitrile gradient from 1 % to 95 % for 105 min in water with a flow rate of 0.2 mL/min. Both solvents contained 0.1 % (v/v) TFA. 100 µL of each digested sample were injected and monitored at 215 nm.

### Example 6: MALDI-TOF spectra of undigested protein

An 18 mg/ml solution of trans-3-indoleacrylic acid in acetonitrile/0.1% trifluoroacetic acid 70/30 (v/v) was premixed with the same volume of sample solution. Then 1.0 µL of the mixture was applied to the target surface. Typically 150 - 200 laser shots were averaged in linear positive ionization mode. The accelerating voltage was set to 25.000 V and the grid voltage to 90%. Bovine albumin M⁺ and M²⁺ were used for external calibration.

### Example 7: SE-HPLC (size exclusion HPLC)

SE-HPLC was performed with a Waters Alliance 2690 HPLC system equipped with a TosoHaas TSK gel G 4000 SWXL column (7.8 x 300 mm). Proteins were eluted using a mobile phase containing 0.02 M NaH₂PO₄, 0.15 M NaCl, 1 % (v/v) diethylene glycol and 10 % (v/v) ethanol (pH 6.8) at a flow rate of 0.4 mL/min and detected at 210 nm. The injection amounts were 20 µg of each isomers.
Size Exclusion HPLC and SDS-PAGE were used to determine the amount of oligo-PEG-IFN alpha 2a forms and aggregates in the different IEC fractions. The reference material contains 2.3 % aggregates and 2.2 % oligomers.
Peaks 1, 4, 4a, 5, 6 and 8 contain < 0.7 % of the oligopegylated IFN alpha 2a forms, whereas in peaks 2, 3, and 7 the percentage of the oligopegylated IFN alpha 2a forms are under the detection limit (< 0.2 %). In the case of the aggregates a different trend could be seen. In all peaks the amount of aggregates is below 0.9 %.

### Example 8: SDS-PAGE

SDS-PAGE was carried out both under non-reducing and under reducing conditions using Tris-Glycine gels of 16 % (1.5 mm, 10 well). Novex Mark 12 molecular weight markers with a mass range from 2.5 to 200 kDa were used for calibration, bovine serum albumin (BSA) was used as sensitivity standard (2 ng). Approximately 1 µg of all the samples and 0.5 µg of standard were applied to the gel. The running conditions were 125 V and 6 W for 120 min. The proteins were fixed and stained using the silver staining kit SilverXpress from Novex.

The gels that were recorded under non-reducing conditions for the IEC fractions 1 - 8 show a pattern that is comparable to that of the PEG-IFN alpha 2a reference standard. Under reducing conditions, the gels show an increase in intensity of the minor bands at about 90 kDa as compared to the standard. Between 6 and 10 kDa protein fragments appear for peaks 6, 7 and 8. Both bands together correspond to approximately 1 % of clipped material. In the lanes of isomer 1, 5, 6, 7, 8 additional bands with more than 100 kDa can be seen which are also present in the standard. These can be assigned to oligomers. Thus SDS-PAGE confirms the results of the SE-HPLC analysis.

Overall, RP-HPLC and SDS-PAGE experiments indicate that the purity of the IEC fractions can be considered comparable to the PEG-IFN alpha 2a reference standard.

The structure of the PEG-IFN alpha 2a species derived from the 9 IEC-fractions were identified based on the results of the methods described above using the strategy mentioned above.

### Example 9: In Vitro Antiviral Protection Activity

The antiviral activities of the isolated isomers were determined by the reduction of the cytopathic effect of vesicular stomatitis virus (VSV) on Madin-Darby bovine kidney (MDBK) cells. The cell lines were cultured in MEM-medium containing 10% fetal bovine serum. The isomers and PEG-IFN were diluted to a final concentration of 10 ng/ml (assay starting concentration) for the detection of the effective concentration. This effective concentration (EC₅₀) was determined as the concentration needed for 50% protection of the cells against the VSV. In all measurements, the cell culture was challenged with VSV 4 hours after addition of the PEG-IFN isomers. After 18 hours growth at 37°C the cells were stained with crystal violet® to determine the number of intact cells.

To determine the antiviral activity of the 9 positional isomers the EC₅₀-values were measured. PEG-IFN was tested in this assay as a reference.

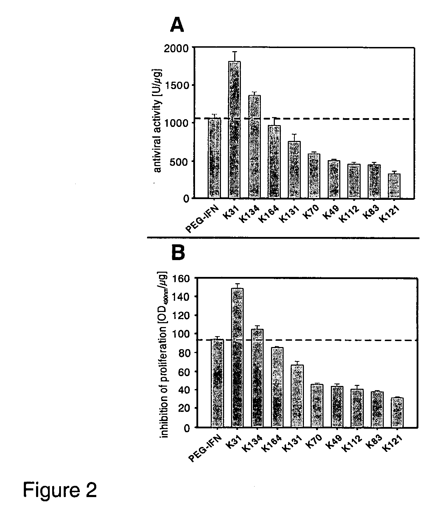

Figure 2A shows the specific activities (U/µg, grey columns) of the isomers. In this assay all positional isomers had activity, however at a different level. The isomers K31 with 170% and K134 with 125% display higher values than PEG-IFN. K164 was equal to the mixture whereas the activities of K49, K70, K83, K112, K121 and K131 were lower.

### Example 10: Anti-proliferative Activity Assay.

For this assay, a primary cell culture of human melanoma metastases cells (ME15) was used. This cell line was deposited on August 17, 2000 under the terms of the Budapest Treaty at the Collection Nationale de Cultures de Microorganismes, Institut Pasteur, Paris under the registration number CNCM 1-2546. The cell line was cultured in RPMI medium supplemented with 10% FCS, 2 mM glutamine, 1mM sodium pyruvate, 10 mM non-essential amino acids and HEPES buffer (all from Gibco Life Sciences, UK). When confluent, the cells were passaged by trypsinization. Approximately 5000-6000 cells per well in a flat bottom 96 well plate were used as a start point for the cell proliferation assay in the presence or in the absence of the individual concentrations of IFN, PEG-IFN and the isomers over a period of a 5 day period. The number of living cells was then determined using a cell staining kit (Promega, Madison, USA) based on the colorimetric detection of the cleavage of the tetrazolium salt MTS into formazan. The MTS reaction solution was added according to the manufacturer's recommendation for a period of 4 hours at 37°C, after this period the absorption at 490 nm was recorded in a spectrophotometer.

The specific anti-proliferative activity of the isomers was determined by using an IFN- sensitive melanoma cell line (ME15) and a commercial assay. Positional isomers and PEG-IFN as references were used at a concentration of 100 U/ml (Figure 2B). The biological unit (U) for the isomers which was used for the experiments was defined and determined in the antiviral assay hereinbefore described, with PEG-IFN as standard reference.

In this assay all isomers were active with the isomer K31 at 159% and K134 at 115% relative to PEG-IFN. K164 displayed the same activity as PEG-IFN; all other isomers were less active than the reference.

The results rank the activity essentially in the same order as in the antiviral assay indicating that same activities are derived from a common trigger or signaling event.

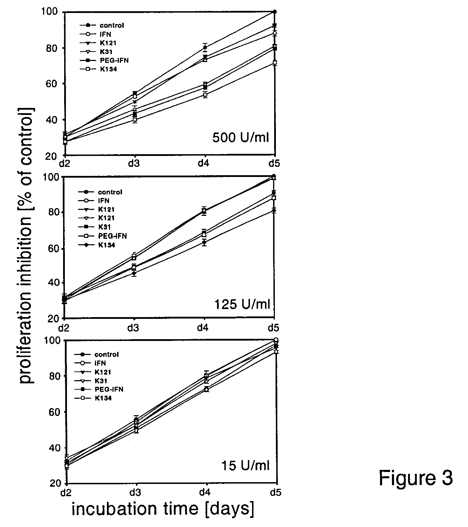

One explanation for the different specific activities of the nine positional isomers in the two previous experiments could be their different protein stability. To find this out we tested some isomers at different concentrations in a time course experiment using the ME15 cell line. We selected isomers K31 and K134 which have the highest activity, and K121, which is the isomer with the lowest activity, together with IFN and PEG-IFN as references (see Figure 3). Different concentrations of IFN, PEG-IFN and positional isomers (500, 125 and 15 U/ml) were used for the treatment of the ME15 cells over a period of 5 days. Every 24h the number of cells was measured.

Again we found a striking correlation to the anti-proliferative activity described above with K134 being most active followed by K31 and PEG-IFN. As expected the lowest activity was found for K121 and IFN.

It can be observed that the inhibition of proliferation is decreased by diminishing concentration. Interestingly, PEG-IFN used at a concentration of 500 U/ml exhibits identical anti-proliferative activity as compared to the isomer K134 used at 125 U/ml after 5 days, while IFN and K121 exhibit lower or no anti-proliferative activity after 5 days when used at a concentration of at least 125 U/ml.

In three independent biological assays we established a correlation between the intramolecular attachment site of the PEG moiety and the biological activity. In summary, the isomers K31 and K134 showed consistently higher specific activities than the commercial mixture, while the remaining seven isomers had a similar specific activity compared to the commercial mixture (K164). Activities range between 170% and 20% indicating that the difference in specific activities of the protein between the positional isomers is significant.

### Example 11: Expression and purification of the recombinant extracellular domain of the Interferon Receptor 2 from E. coli:

The extracellular domain of the Interferon Receptor 2 (IFNR2-EC) was cloned into a pET15b-vector (Invitrogen, US) with an N-terminal His-Tag®. This vector construct was used to express IFNR2-EC in *E.coli.* The target protein precipitated in the cells in the form of inclusion bodies. The *E. coli* pellet after the fermentation in full medium was resuspended in lysis buffer containing 50 mM Tris, 10% saccharose, 0.02% sodium azide, 10 mM magnesium chloride and EDTA-free protease inhibitor. After the homogenization in a French Press and centrifugation (13000 rpm) the inclusion bodies were solubilized in 8 M urea. The first chromatography step was a nickel chelate chromatography with a linear gradient from 0-500 mM imidazol over 10 column volumes. The pooled peak of IFNR2 was dialyzed against 8 M urea to remove the imidazol. After this dialysis the sample was diluted to 0.1 A₂₈₀ with 8 M urea containing 150 mM Tris/HCL pH 8.0 and stored at room temperature until the Ellman assays were negative. The oxidized IFNR2-EC was dialyzed against 50 mM Tris pH 8.4 for refolding. 50 mM Tris pH 8.4 was also the starting buffer for the second chromatography. The second chromatography step was with an anion ion-exchange resin (HiTrap Q HP, 5×5ml, Amersham Biosciences) with a gradient of 0-500 mM NaCl.

All purification steps were analyzed by SDS-PAGE under reduced and non-reduced conditions. The protein purity was monitored by analytical size-exdusion chromatography using Superdex® 75 and 200 HR (PC 3.2/30, Amersham Biosciences). This chromatography method was also used to determine both the binding activity and capacity (data not shown).

### Example 12: Characterization of the interaction of IFN and the PEG-IFN isomers with the IFNR2-EC using an optical biosensor system:

The immobilization of the IFNR2-EC was achieved by labeling with biotin. The biotinylated protein was anchored to a streptavidin sensor surface (SA Sensor, BIAcore).

Biotinylation was performed by diluting the protein stock solution (10 mM acetate, pH 7.5) with Hepes (10 mM, pH = 8.4) to a final protein concentration of 0.23 mg/ml protein (8.65 µM). A 8.65 mM solution of biotinamidocaproate-n-hydroxysuccinimide ester (Fluka) in DMSO was added to the solution to give a final concentration of the biotinylating reagent of 86.5 µM. The mixture was allowed to react for 1h before it was dialyzed over night against a Hepes buffer (10 mM, pH 8.4, 150 mM NaCl) to remove low molecular weight reaction products. This solution was used in the immobilization procedure. Immobilization was achieved by contacting the solution of the biotinylated protein with the SA sensor surface. The amount of immobilized protein was controlled via the contact time. For the binding experiments 4000 RU, corresponding to approximately 4 ng/mm² of protein were immobilized.

Binding experiments were performed in Hepes buffer (10 mM Hepes, 150 mM NaCl, 3 mM EDTA, 0.005% polysorbate 20, pH 7.4) as the running buffer. Interferon solution was prepared by diluting stock solutions of the proteins with running buffer. The flow velocity was set to 5 µl/min for all binding experiments. The interferon solutions were contacted for a time interval of 5 min with the immobilized IFNR2-EC.

Calculation of binding data: dissociation constant (K_{D}) values were determined by fitting the time dependent response curves with a mathematical fit based on the simple kinetic model of the formation of a 1/1 complex. kₒₙ and k_{off} values were obtained from this fit. K_{D} represents the ratio k_{off}/kₒₙ

Investigation of the interaction of proteins by label-free solid phase detection such as surface plasmon resonance (SPR) requires the immobilization of one of the interacting compounds.

We used two different coupling strategies to immobilize IFN or IFNR2-EC to the sensor surface. The IFN was immobilized via free amino groups on a carboxylated dextran sensor surface, the IFNR2-EC was labeled with biotin and anchored to a streptavidin sensor surface. With these two sensors and the corresponding IFN respectively IFNR2-EC binding test were done. Consistent values of rate constants and binding affinities were obtained. The results indicate that the binding affinity is independent of the immobilization technique and the immobilized protein (data not shown). These preliminary tests were necessary to increase the accuracy for the measurements with the isomers and to verify the simple kinetic model of 1/1 complex formation.

For the determination of the affinity and kinetic constants we selected five isomers (K31, K121, K131, K134 and K164) to cover the whole activity range of the specific biological activity (see Figure 2 A,B). To increase the significance of the kinetic and equilibrium parameters, we performed binding studies on the immobilized IFNR2-EC with different concentrations of the isomers. The IFN and PEG-IFN were used as a reference. The result of the binding curve fits based on a kinetic model for a formation of a 1/1 complex are given in Table 2.

**Table 2:**

| Kinetic constants for the interaction of wild-type IFN; PEG-IFN and several positional isomers | | | |
|---|---|---|---|
| | kₒₙ [10⁵ M-1xs-1] | K_{off} [10⁻² S-1] | K_{Dkin} [nM] |
| IFN | 12.5 | 1.14 | 9.12 |
| PEG-IFN | 0.27 | 2.3 | 851 |
| K₃₁ | n.d. | n.d. | n.d. (> 1mM) |
| K₁₃₄ | 0.87 | 3.96 | 455 |
| K₁₃₁ | 0.96 | 2.8 | 289 |
| K₁₂₁ | 0.83 | 2.33 | 279 |
| K₁₆₄ | 0.652 | 2.19 | 335 |

The table shows the measured affinity and kinetic data. The calculation for the kinetic constant is based on the kinetic model of a 1/1 complex formation. The IFNR2-EC was labeled with biotin. This biotinylated protein was anchored to a streptavidin sensor surface (SA Sensor, Biacor). Binding experiments were performed in Hepes buffer (10 mM Hepes, 150 mM NaCl, 3 mM EDTA, 0.005% polysorbate 20, pH 7.4) as the running buffer.

The obtained K_{D}-values indicate that the pegylation of IFN causes a decrease in the affinity towards the INFR2-EC. The affinity deficit is dependent on the position of pegylation. The affinity change is generally due to a change in the association constants (kₒₙ). For instance, isomer K134 changes the affinity roughly by a factor of 15. kₒₙ is reduced by a factor of more than 10, whereas k_{off} is increased only by a factor of approximately 4.

For the isomer K31 only an estimation of the K_{D} was possible because the protein concentrations were too low.

Combining the data from the specific antiviral or specific anti-proliferative activity with the IFNR2-EC affinity study an interesting correlation was observed as seen in Figure 4. It indicates that the higher the IFNR2-EC affinity (low K_{D}) of a given isomer, the lower the specific biological activity. This correlation is opposed to the common drug screening dogma which is principally used to search ligands which have a low K_{D} and a high biological activity.

### Example 13: Transcriptional response of the isomers compared to IFN

DNA microarrays which permit simultaneous measurement of the expression levels of thousands of genes, provide a comprehensive frame-work to determine how the positional isomers affect cellular metabolism and regulation on a genomic scale.

To estimate the influence of the pegylation site on the transcriptional activity of IFN we measured the IFN-induced gene expression pattern of the melanoma cell line ME15 via the oligonucleotide array technology.

ME15 cells were incubated for 48h with 100 U/ml of IFN, PEG-IFN or PEG-IFN isoforms. After 48h the cultured cells were harvested by scraping and the total cellular RNA was extracted with RNA-Bee™. From each sample 10 µg of the total cellular RNA were reverse transcribed (Invitrogen, U.S.), labeled (Ambion, U.S.) and processed by using commercial kits according to the supplier's instructions. The methods of the alkaline heat fragmentation and the following hybridization of the cDNA with the U95Av2 GeneChip arrays were standard procedure provided by the manufacturer of the microchips (Affymetrix, U.S.). A further example for a procedure for performing oligonucleotide array analysis can be found in Fambrough et al., Diverse signaling pathways activated by growth factor receptors induce broadly overlapping, rather than independent, sets of genes, *Cell* 97 (1999), 727-741.

The raw data of the arrays were measured with a confocal laser scanner (Hewlett Packard, U.S.) and pixel levels were analyzed using GeneChip v3.1 software (Affymetrix, U.S.). The expression level for each gene was calculated as normalized average difference of fluorescence intensity as compared to hybridization to mismatched oligonucleotides, expressed in arbitrary units (AD). Each investigation of the samples was measured in triplicate with labeled cRNA from three different cell plates.

The following four criteria were chosen for differential expression of a gene after treatments with IFN, PEG-IFN or the PEG-IFN isoforms compared to the untreated cells were: (1) The intensity value of the treated cells is at least two fold higher than the untreated cells. (2) The intensity value in the treated cells should be higher or at least 50 AD. (3) The intensity value of the untreated cells should be lower than 50 AD. (4) The standard deviation has to be significantly smaller than the absolute change in average difference and the calculated confidence level of a gene is set greater than 97% (p value < 0.03).

For these experiments we used the same cell line (ME15) as for the anti-proliferative assay. We treated the cells for 48h with 100 U/ml of each isomer or with IFN and PEG-IFN. IFN was used as the reference. After this treatment total RNA was isolated from the ME-15 cells. The methods for preparation of cRNA and subsequent steps leading to hybridization and scanning of the U95Av2 GeneChip Array are described above. Visual inspection of total RNA samples before processing by agarose gel electrophoresis did not reveal any signs of degradation or yield in the samples. The number of biological replicates for each isomer, IFN and PEG-IFN (n=3) allows the application of statistical algorithms for data analysis in order to account for experimental variation. The data analysis focused on genes which were lower than 50 AD in the untreated cells but at least 2-fold up-regulated with an AD value of at least 50 in the treated cells.

The standard deviation had to be significantly smaller than the absolute change in average difference and the calculated confidence level of a gene was set to greater than 97% (p value < 0.03).

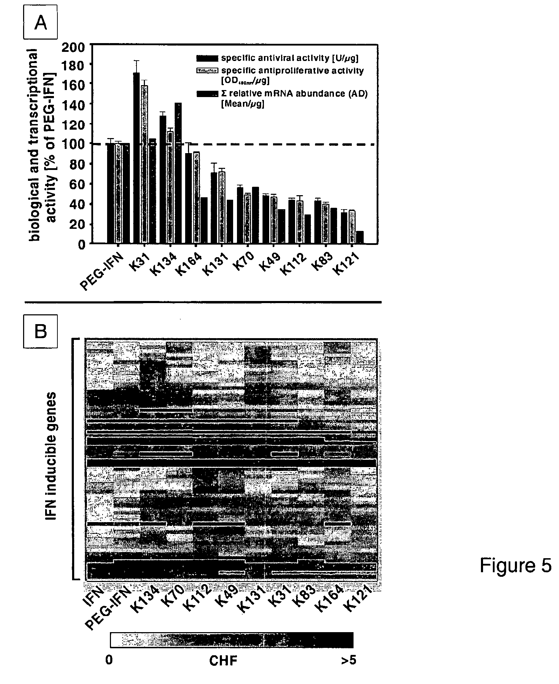

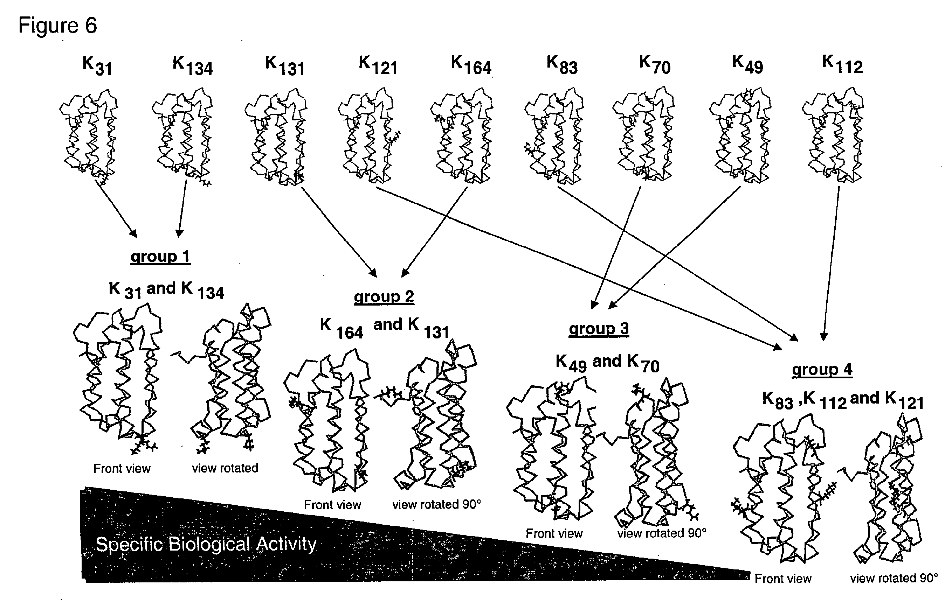

By applying these criteria, an expression profile of 29 up-regulated genes was observed. In this expression pattern 13 known IFN-inducible genes which are involved in the anti-proliferative and antiviral pathway were highly up-regulated as compared to IFN (Table 3). The hierarchical cluster (Figure 5B) illustrates that all isomers have transcriptional activity but generate different intensities.

The sum of AD (∑ AD) of the induced genes was determined for each isoform and IFN, and the transcriptional, antiviral and proliferative activities compared with IFN as a reference. Figure 5 A shows that isoforms with an increased biological activity, as determined by their antiviral and anti-proliferative activities as compared to PEG-IFN, also have an increased transcriptional activity, while isoforms with a lower biological activity display a lower transcriptional activity, and that thus, in contrast to biological activity vs. binding assays, a positive correlation exists between biological and transcriptional activity.

Beside the effect of the isomers on the general transcriptional gene response, a varied up-regulation of individual genes was also observed. For example, the gene for interferon-induced nuclear phosphoprotein shows higher expression upon exposure to K49 and K31 than with K134 or K131. K49 and K31 isomers have a lower general transcriptional gene response than K134 or K131. This observation emphasizes the necessity of analyzing the general transcriptional gene response, as determined by the method of this invention, rather than to measure transcriptional expression levels of an individual gene, or a small selection of genes, for the determination of the transcriptional activity of a compound of interest.

The data of the microarray chips were also analyzed for transcriptional-specific side-effects which may indicate undesirable effects on the metabolism or vitality of cells by any isomer. The criteria for these analyses were similar to those described before, the only difference was that the data for each isomer were cross-compared. The result of this analysis showed no specific side-effect of any positional isomer.

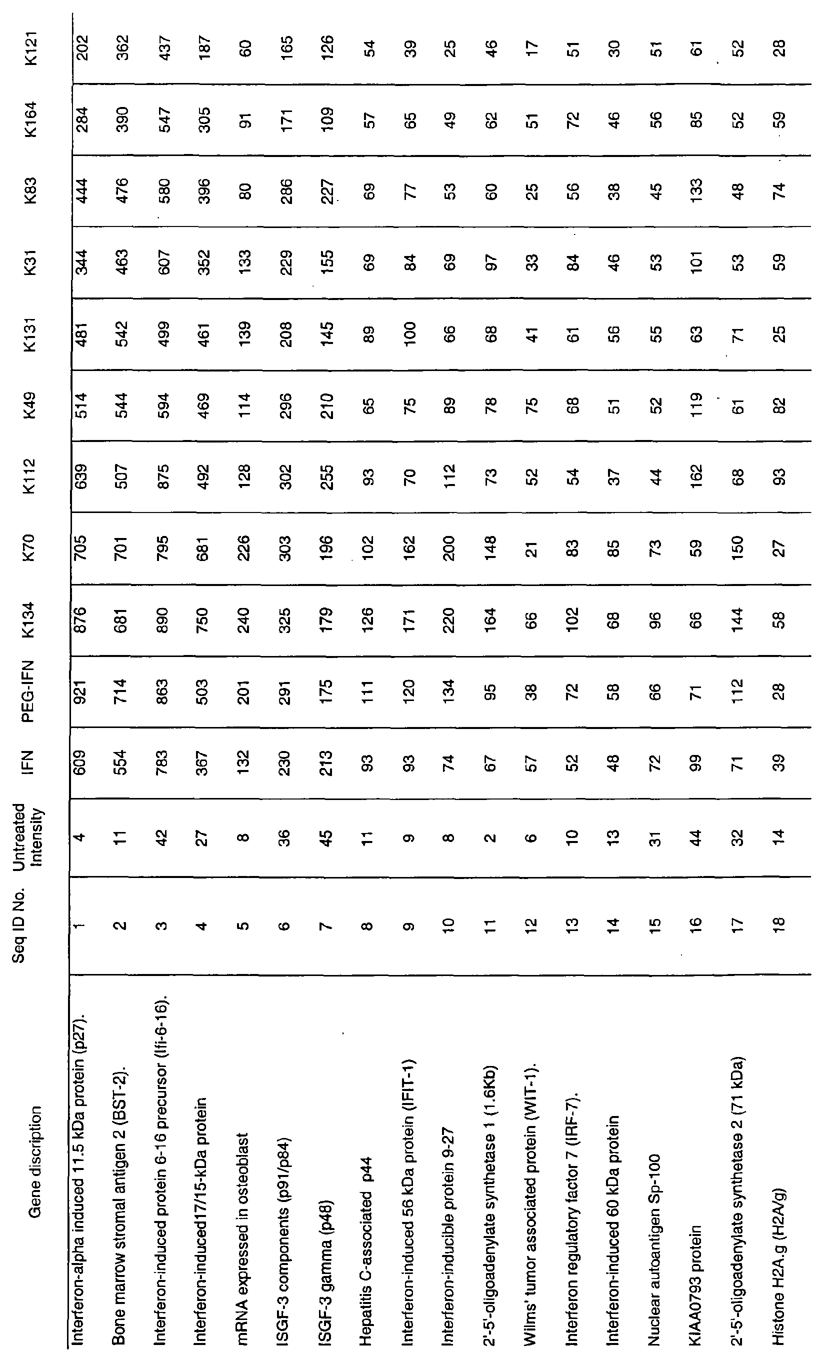

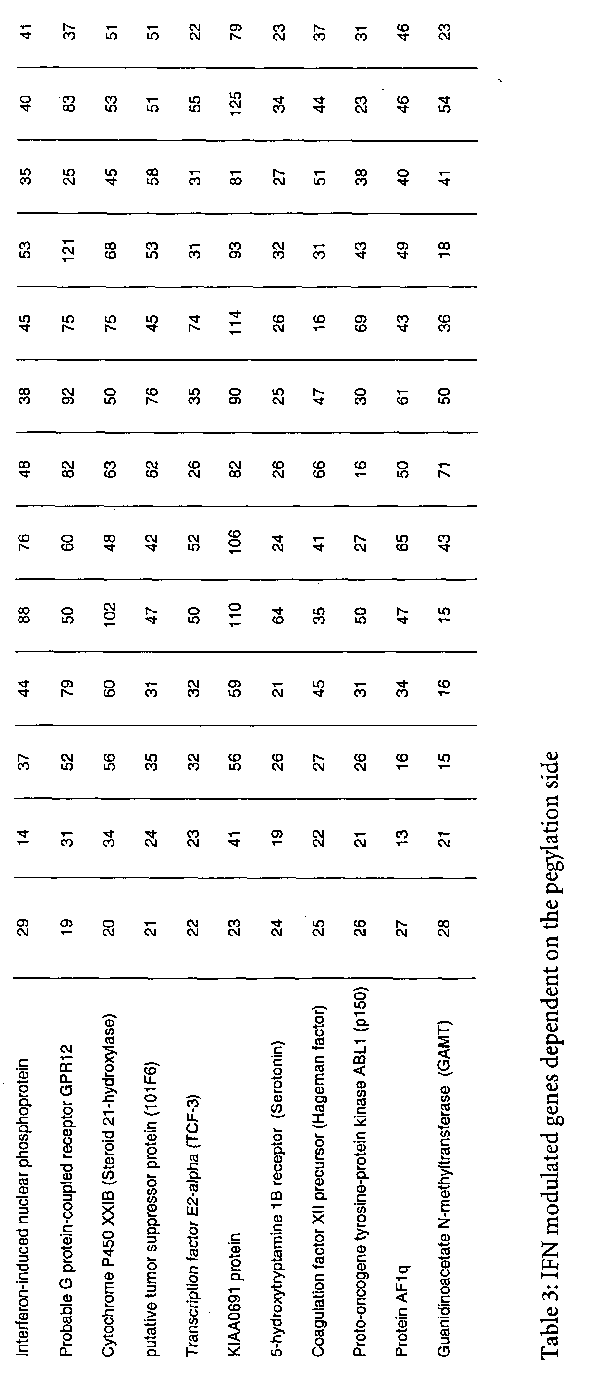

To identify induced genes the ME15 cell line were cultured 48 hours in absence (untreated cell) or in presence of 100 U/ml IFN or a PEG-IFN sample. The expression level for each gene was calculated as normalised average difference of fluorescence intensity as compared to hybridisation to mismatched oligonucleotide, expressed in arbitrary units. Each investigation of the samples were measured in triplicate with labelled cRNA from three different cell plates. The criteria for a differentially expression of a gene by treatments with IFN or a PEG-IFN sample compared to the untreated cells were: if the mean value increase was at least twofold higher as in the untreated cells and the main value in the treated cells should be higher or at least 50 units. The mean value of the untreated cells should be lower than 50 units. In addition, the standard deviation had to be significantly smaller than the absolute change in average difference and the calculated confidence level of a gene was set greater than 97% (p value < 0.03). By applying these criteria, 29 genes were found to be modulated.

## Claims

1. A method for determining the biological activity of a compound which can modulate gene transcription, comprising the steps of
a) contacting a host with a compound;
b) determining the general transcriptional gene response of the host; and
c) quantitating the general transcriptional gene response induced by said compound.

2. The method of claim 1, wherein the general transcriptional gene response is determined by a DNA array technology.

3. The method of claim 2, wherein the DNA array technology is an oligonucleotide array technology.

4. The method of any one of claims 1 to 3, wherein the compound is a modified compound.

5. The method of claim 4, wherein the modified compound is a modified protein.

6. The method of claim 5, wherein the modified protein is a pegylated protein.

7. The method of claim 6, wherein the pegylated protein is a specific isolated isoform of a pegylated protein.

8. The method of claims 6 or 7, wherein the pegylated protein is Epo or INF.

9. The method of any one of claims 1 to 8 wherein the transcriptionally regulated genes are one or more genes selected from the group consisting of Seq ID No. 1 to 29.

10. A method for determining the relative biological activity of a modified compound, comprising measuring the biological activity of the modified compound and the respective unmodified compound by the method of any one of claims 1 to 7, wherein the relative transcriptional activity of the modified protein is determined by calculating the ratio of the biological activity of the modified compound and the biological activity of the unmodified compound.

11. The method of claim 8, wherein the modified compound is a chemically or enzymatically modifed compound.

12. The method of claim 9, wherein the modified compound is a pegylated protein.

13. A method for identifying compounds that indirectly modulate gene transcription comprising determining the biological activity of a first compound which is a known modulator of gene transcription by the method of any one of claims 1 to 9 in the presence or absence of a second compound, wherein a compound which indirectly modulates gene transcription is identified if the biological activity of the first compound measured in the presence of said second compound is significantly different from the biological activity of the first compound measured in the absence of said second compound.

14. The method of claim 13 wherein the known modulator of gene transcription is Epo or IFN.

15. A compound identified by the methods of claims 1 to 14 which modulates gene transcription.

16. Use of the method of claims 1 to 14 for identifying a compound which modulates gene transcription.

17. A kit comprising components for carrying out the methods according to claims 1 to 14.

18. The kit of claim 17 wherein said components comprise a DNA array.

19. A pharmaceutical composition comprising a compound of claim 15 and a pharmaceutically acceptable carrier.

20. Use of a compound of claim 15 or a pharmaceutically acceptable salt thereof for the preparation of a medicament for the treatment of a disease, preferably a viral disease or of a cancer.

21. The methods, compounds, kit and uses as hereinbefore described, especially with reference to the foregoing examples.


SEQUENCE LISTING

<110>  F. Hoffmann-La Roche AG

<120>  Transcriptional Activity Assay

<130>  21574

<160>  29

<170>  PatentIn version 3.1

<210>  1

<211>  597

<212>  DNA

<213>  Homo sapiens

<220>

<221>  interferon-alpha inducible gene; ISG12 gene

<222>  (1)..(597)

<223>  GenBank accession No. X67325

<400>  1

agctgaagtt gaggatctct tactctctaa gccacggaat taacccgagc aggcatggag      60

gcctctgctc tcacctcatc agcagtgacc agtgtggcca aagtggtcag ggtggcctct     120

ggctctgccg tagttttgcc cctggccagg attgctacag ttgtgattgg aggagttgtg     180

gccatggcgg ctgtgcccat ggtgctcagt gccatgggct tcactgcggc gggaatcgcc     240

tcgtcctcca tagcagccaa gatgatgtcc gcggcggcca ttgccaatgg gggtggagtt     300

gcctcgggca gccttgtggg tactctgcag tcactgggag caactggact ctccggattg     360

accaagttca tcctgggctc cattgggtct gccattgcgg ctgtcattgc gaggttctac     420

tagctccctg cccctcgccc tgcagagaag agaaccatgc caggggagaa ggcacccagc     480

catcctgacc cagcgaggag ccaactatcc caaatatacc tgggtgaaat ataccaaatt     540

ctgcatctcc agaggaaaat aagaaataaa gatgaattgt tgcaactctt aaaaaaa        597

<210>  2

<211>  996

<212>  DNA

<213>  Homo sapiens

<220>

<221>  BST-2

<222>  (1)..(996)

<223>  GenBank accession No. D28137

<400>  2

gtggaattca tggcatctac ttcgtatgac tattgcagag tgcccatgga agacggggat      60

aagcgctgta agcttctgct ggggatagga attctggtgc tcctgatcat cgtgattctg     120

ggggtgccct tgattatctt caccatcaag gccaacagcg aggcctgccg ggacggcctt     180

cgggcagtga tggagtgtcg caatgtcacc catctcctgc aacaagagct gaccgaggcc     240

cagaagggct ttcaggatgt ggaggcccag gccgccacct gcaaccacac tgtgatggcc     300

ctaatggctt ccctggatgc agagaaggcc caaggacaaa agaaagtgga ggagcttgag     360

ggagagatca ctacattaaa ccataagctt caggacgcgt ctgcagaggt ggagcgactg     420

agaagagaaa accaggtctt aagcgtgaga atcgcggaca agaagtacta ccccagctcc     480

caggactcca gctccgctgc ggcgccccag ctgctgattg tgctgctggg cctcagcgct     540

ctgctgcagt gagatcccag gaagctggca catcttggaa ggtccgtcct gctcggcttt     600

tcgcttgaac attcccttga tctcatcagt tctgagcggg tcatggggca acacggttag     660

cggggagagc acggggtagc cggagaaggg cctctggagc aggtctggag ggccatgggg     720

gcagtcctgg gtgtggggac acagtcgggt tgacccaggg ctgtctccct ccagagcctc     780

cctccggaca atgagtcccc cctcttgtct cccaccctga gattgggcat ggggtgcggt     840

gtggggggca tgtgctgcct gttgttatgg gttttttttg cggggggggt tgcttttttc     900

tggggtcttt gagctccaaa aaataaacac ttcctttgag ggagagcaaa aaaaaaaaaa     960

aaaaaaaaaa aaaaaaaaaa aaagaattcc accaca                               996

<210> 3

<211> 6803

<212> DNA

<213> Homo sapiens

<220>

<221> interferon-inducible peptide (6-16) gene

<222> (1)..(6803)

<223> GenBank accession No. U22970

<400> 3

agatctatca tgatggccac atgaacacag gcttcactgg tcttaccata tgcccatgac 60

ccagaagcag ccagcctgag agaacaatgg aacaaagtga cttaatttcc aaagttctgg 120

gggtttataa acagcatatg gtatgtcaca tgactactgt gggattggaa aaagatcatg 180

taaatagagc actttgcaca gaccctggca tgcagcaagt gctcaataaa tgataggtgt 240

tgtttactaa ttggactgaa tggtgaaagg cctgtgtgcc ccagggggag ctggtgatca 300

ggcttcacta agcccagtat ggccgtggct ctcatctcag tgtgacatgc tttgaatacc 360

cttagcggct ccaaaagtcc tcagcttgaa gtgcattttt ctgccagcag gcagcacaca 420

aatgttccgc tgggcggagc tgggagagag gggaaaatga aactctgcag agtgcaggag 480

ctgggagaga ggggaaaatg aaactgcaga gtgcagaaat agaaactccg acagggattg 540

gctgcctagg gtgagacgtg ggaggatcca caagtgatga taaaaagcca gccttcagcc 600

ggagaaccgt ttactcgctg ctgtgcccat ctatcagcag gctccgggct gaagattgct 660

tctcttctct cctccaaggt aaactcagga gcttatgaag tgtgggcatt caagctgcca 720

ccctctgcca ggctgcctgt ctgcctgtaa atctcatgtt ctgagagcca ggaggcccct 780

tctcctggga ggcagcactc ctgggtccct tttagtgctc tgggctggga cttgtctaag 840

aggatgggtt ggagattttt agggagatgg gatgcaaaac cccaagtggc atgagaccca 900

gcttacaggt gcaatatcag cgatctgtgg ccttaacact gtcacctctt ggagccttaa 960

ttacttcctc tgtaaaagga aagttaagtt gcctttgctg ctcaaaggac tggagtattt 1020

tagaatcccc tttattatag gatccaatgt gacagtgagt tcatctttaa ggatagatga 1080

agccatctgg agtggttgtt aaaaatgtgg gcttggtggc caggcgtggt ggttcacgcc 1140

tgtaatccca gcactttggg aggccgagac ccacggatca cgaggtcagg agatcgagac 1200

catcctggct aacacagtga aaccctgtct ctactaaaaa tacaaaaaaa attagccggg 1260

cttggtggtg ggcgcctgta gtctcagcta ctcgggaggc tgaggcaggc aggagaatgg 1320

catgaacctg ggaggtggag cttgcagtga gatgagattg cgccactgca ctccagcctg 1380

ggcgacagag aaagactcca tctcaaaaaa aaaaaaaaaa aaaatgtggg cttgggtatt 1440

agccaattgt gggtttaaac cctgactttg ccatgacctg gctaggcagc cttgggaata 1500

ttacttaacc tctctgagct tcagtttcct tatctataac atgtggttga taacaatggt 1560

acctacttcg tggtgttgtt ataggtacaa gtggactgcg tgccataaag tgctaagtac 1620

tcaatacatg agaggagttt cttgtttttc tttttaactt aagaaaatag cattgatctg 1680

acaaagatca tattagaggt gagtctgagg gcatatgcat tttccagttt tgtggtataa 1740

aggaaagagc ataggttcat gaattctgac tgtcttgaat gtgtacccat gttgcacaat 1800

ttgctgtgtg acctttatcc cttgtgatat tgtaaaatat attttttcat cccccagttc 1860

ctggcataca actcctaaaa tccttggaat cttcaaagtg ataaatatct tttgagtgat 1920

aactgtctaa taagttgact gatggctggc atttcctctg ggagggggag aggggctgaa 1980

ggttaagctg attaccaatg ggcagtgatt taatcagtca tgcctatgta atgaagcctc 2040

cataaaaaca caaaaggaca gggtttgaca agcttccaga tagccgaata caggaggtct 2100

ccagtgggtg gtgcttctgg agaggaatgg aaactccatg ccctttctcc cataccttgc 2160

cctatgcatc tcttcatctg gagcatttgt aatatccata aggaaccagt aaatgtagct 2220

gggtatggtg gctgatgcct gtaatctcag cactttggga ggctgaggca ggcagattgc 2280

gtgagttcag gagttcaaga tcagcctggg caacatggtg aaaccccatc tcaaaaaaaa 2340

aaaaaaaaat tagttgggcg tggtggtgca tacctgtagt cccagctatc tggaggctga 2400

ggtgggagga tcacttgagc ctgggaggcg aaggtctcag tgagctgaga tggggccact 2460

gcactccagc ctgagcaata gagtgagacc ctgtctcaaa acaacaacaa gatcaatttt 2520

tttttttaa agaaagaaac cagtatatgt aagtaaagtg tttccctgag ttctgtgagc 2580

tgctctagca aattagtaaa actgcaggag ggaattgtgg gaaccccagt ttatagtcag 2640

tcagaaacac aggtaaaaca acctagggct tcgattggca gtctggggga ctgaaccctc 2700

aacatgtggg atcggccact atctacccat agtggtgtca gaattgaatt tggggaccct 2760

cagctggtgt tcaccagagg actgattgtt tgcttgctgg tggggagaaa cccccacact 2820

tctgctgtca gaagcctctc gtacggtgag ggaaactgag tttctttttc cacacaccct 2880

tctgtgcttt ctttctgaaa aaaaaattaa gttgatgtga ggccaacgtt tggtgccagt 2940

agatattcaa tgcattgtaa gaatgagttt gtcttaaaaa tttttttaaa gcaaattcta 3000

tgcgtcaggc actgttcaaa gtgcgggata cagcgtggaa cagttattaa attaacaagt 3060

accctctgaa atagactcta ttaatacccc aattttagaa ataaacaaac tgaagcccag 3120

agaagcaaag taccctagta tcctgcccaa ggtcacttgg gtacaaggcc aagaacaacc 3180

acagtcatga tattgacatc tgcggacgtt accacgtgcc aaacaccgtg cgaatccgca 3240

cagaatccgc acacccaccg gttggggcag ggacatctct gccactgatg cggagaggaa 3300

acggattccc gaggctcctg agagggatcc tgggttccat tcaccacacc tcgctgcttt 3360

ttactgattc agttggaact ggaagggaaa acaacggagc tgatgagctc caaaaatgct 3420

ccggggacca tctccctctc gcccgttcgc aggtctagtg acggagcccg cgcgcggcgc 3480

caccatgcgg cagaaggcgg tatccgtttt cttgtgctac ctgctgctct tcacttgcag 3540

tggggtggag gcaggtgaga atgcgggtaa ggatgcaggt aaggggacag gtaagggtgc 3600

aggtaaggat gcaagtaagg gtgcaggtaa ggatgcgggt aaggatgcag gtaaagatgc 3660

gggtaaggat gcaggtaagg atgcgggtaa ggatgcaggt aagggtgcag gtaaggatgc 3720

gggtaaggat gcaggtaagg atgcaggtaa gggtgcaggt aaggatgcag gtaaggatgt 3780

gggtaaggat gcaggtaagg gtgcaggtaa gggaacagat aagggtgcag gtaaggggac 3840

aagtaaggag gcatataagg gtgctggggg atgggaggag tgtgggataa aggagagagt 3900

ttcagggtcc ggggtgtaga gggtctggcg tgcttctgta gagaccctgg cgtccattcc 3960

aatggacagc gcagggttgg tgtcgccaga gtcccagatt caaatcccac tactctgttc 4020

attcctccat cctaccgaaa tttactcgat gctagttctg tgccagctcg gcgcctaggg 4080

gaaagtccct tcccctctgt gaggctctct gagtaggaga gtggtggccc ccgcctccag 4140

gagtggtcct gagcatcaga cacagagtag gggacccctg tgtcccaccc caacaacccc 4200

actacgcttt gtctgctctc ctgcaggtaa gaaaaagtgc tcggagagct cggacagcgg 4260

ctccgggttc tggaaggccc tgaccttcat ggccgtcgga ggaggtgggt ctggagggcg 4320

aggatctcgg gcaggcgggg cgggcctctg ccgcggacgc tccctcacct gctcctgttc 4380

ctccaggact cgcagtcgcc gggctgcccg cgctgggctt caccggcgcc ggcatcgcgg 4440

ccaactcggt ggctgcctcg ctgatgagct ggtctgcgat cctgaatggg ggcggcgtgc 4500

ccgccggggg gctagtggcc acgctgcaga gcctcggtga gtgcggggcc tgggcctggt 4560

gggacgttct ttactattat tttcatcata catctgggga aactgaggca ctcaggaatt 4620

aagtaattta ctcaagtaat taagtagttt accaggaatt aagtgattct ttatttattt 4680

attttgagac agagtctggc tgtgtcaccc aggcaggctg aagtgcagtg gtgtgatctt 4740

ggctcactgc aacctctgcc tccctggctc aagcgatttt cctgccacag cctcccaagt 4800

agctgggatt acaggcacac gccaccacgc ccggctaatt tttgtatttt tagtagagat 4860

ggggtttcac catgttggcc aggctggctc aaactcctga cctcagctga tccgcccgcc 4920

tcggcgtccc atagtgctag gattacaggc gtgagtcacc gcgtccggcc agaaattaag 4980

taacttactc aagtctccct gctagtttag accagagcct agattctgac ccagtcagta 5040

caatgcgaga agccccattc tcagcctcaa aggctgggtg cctcactgat gctttggcca 5100

aatcacaagc ctcagttccc tcagcggtgc gtggggatga tcatcctcag tttaacattt 5160

tttgaatctg ctcaatctca gagaccacag tagcaaagaa acatagtaga gtaactcaaa 5220

tcaatctcac accaaactgg tcttgacttg cactttttga acaccatgga gggaacagat 5280

aacagagctc acagtgactc ttctatcgat tgatcaatct attgatctat ttattataga 5340

taaaggtcaa aacaaggtct cacactgcca ggttggaatg cagtgcatga tcacagctca 5400

cagcagcctc gaactcccag gttcaagcta tcttcctgcc tcagattccc aagtagctgg 5460

gactataggc gcataccgcc acacactggc tttttttttt tttttttttt ttttttttgt 5520

agagacgggg tctcattgtg ttgcccagga tggtcttgaa ttcctgggct caagcaatcc 5580

tcccactttg gcctcccaaa gtgttgagac tgcaagcatg agccactgtg ctggcccaga 5640

gtgactcata aaaaatggcc ttacttccct ctctctcctc tctccccacc cacccccacc 5700

tctctctcgc gctctgaggc ctccaaaatc ctggagaaaa cctgcccctg acaaacttcc 5760

ctctctgcct ttctgaacct cgcatctcct cttctctcaa ttctgaatgg caaaagccca 5820

aagaaccagc ccaaaagaag agagccctgc tcagacgggg ccacacccct gcaatgggag 5880

gggaagagtg tgggcgagcc cagggacacc tctgggctat cagctagttg tcctctcaca 5940

tggcacatag ctgtgacaag atgaaatgat ccctggcttg tgaacccccc acacccacac 6000

agggctccat gaaggtggct gttagcatga ttaataacat ggcgtttcca tggcagacat 6060

caggtgaagg agccattatt tttgcctttt tcatcactga aacacatctc tgatttggga 6120

tgtcaaggca aggtaaaaat gggactcact ttgtagtagt gccatcccct ctcagggcct 6180

ctgtttctcc atctagacat aaggggttgg actcaaccaa tggtttccag acatttggat 6240

tcccaggcct ggcacttggg cccagtgact catctgtgtt tgctttacag gggctggtgg 6300

cagcagcgtc gtcataggta atattggtgc cctgatgcgg tacgccaccc acaagtatct 6360

cgatagtgag gaggatgagg agtagccagc agctcccaga acctcttctt ccttcttggc 6420

ctaactcttc cagttaggat ctagaacttt gccttttttt tttttttttt ttttttttga 6480

gatgggttct cactatattg tccaggctag agtgcagtgg ctattcacag atgcgaacat 6540

agtacactgc agcctccaac tcctagcctc aagtgatcct cctgtctcaa cctcccaagt 6600

aggattacaa gcatgcgccg acgatgccca gaatccagaa ctttgtctat cactctcccc 6660

aacaacctag atgtgaaaac agaataaact tcacccagaa aacactttgt cctgctgtca 6720

atcatgtttg cagtgagaag cccaaaacaa tctggctctg gcctgcacca tccacacacc 6780

cccattccca ttttcccctc gag 6803

<210> 4

<211> 634

<212> DNA

<213> Homo sapiens

<220>

<221> interferon-induced 17-kDa/15-kDa protein

<222> (1)..(634)

<223> GenBank accession No. M13755

<400> 4

cggctgagag gcagcgaact catctttgcc agtacaggag cttgtgccgt ggcccacagc 60

ccacagccca cagccatggg ctgggacctg acggtgaaga tgctggcggg caacgaattc 120

caggtgtccc tgagcagctc catgtcggtg tcagagctga aggcgcagat cacccagaag 180

attggcgtgc acgccttcca gcagcgtctg gctgtccacc cgagcggtgt ggcgctgcag 240

gacagggtcc cccttgccag ccagggcctg ggccctggca gcacggtcct gctggtggtg 300

gacaaatgcg acgaacctct gagcatcctg gtgaggaata acaagggccg cagcagcacc 360

tacgaggtcc ggctgacgca gaccgtggcc cacctgaagc agcaagtgag cgggctggag 420

ggtgtgcagg acgacctgtt ctggctgacc ttcgagggga agcccctgga ggaccagctc 480

ccgctggggg agtacggcct caagcccctg agcaccgtgt tcatgaatct gcgcctgcgg 540

ggaggcggca cagagcctgg cgggcggagc taagggcctc caccagcatc cgagcaggat 600

caagggccgg aaataaaggc tgttgtaaga gaat 634

<210> 5

<211> 2058

<212> DNA

<213> Homo sapiens

<220>

<221> mRNA expressed in osteoblast

<222> (1)..(2058)

<223> GenBank accession No. AB000115

<400> 5

gcacgaggaa gccacagatc tcttaagaac tttctgtctc caaaccgtgg ctgctcgata 60

aatcagacag aacagttaat cctcaattta agcctgatct aacccctaga aacagatata 120

gaacaatgga agtgacaaca agattgacat ggaatgatga aaatcatctg cgcaactgct 180

tggaaatgtt tctttgagtc ttctctataa gtctagtgtt catggaggta gcattgaaga 240

tatggttgaa agatgcagcc gtcagggatg tactataaca atggcttaca ttgattacaa 300

tatgattgta gcctttatgc ttggaaatta tattaattta cgtgaaagtt ctacagagcc 360

aaatgattcc ctatggtttt cacttcaaaa gaaaaatgac accactgaaa tagaaacttt 420

actcttaaat acagcaccaa aaattattga tgagcaactg gtgtgtcgtt tatcgaaaac 480

ggatattttc attatatgtc gagataataa aatttatcta gataaaatga taacaagaaa 540

cttgaaacta aggttttatg gccaccgtca gtatttggaa tgtgaagttt ttcgagttga 600

aggaattaag gataacctag acgacataaa gaggataatt aaagccagag agcacagaaa 660

taggcttcta gcagacatca gagactatag gccctatgca gacttggttt cagaaattcg 720

tattcttttg gtgggtccag ttgggtctgg aaagtccagt tttttcaatt cagtcaagtc 780

tatttttcat ggccatgtga ctggccaagc cgtagtgggg tctgatacca ccagcataac 840

cgagcggtat aggatatatt ctgttaaaga tggaaaaaat ggaaaatctc tgccatttat 900

gttgtgtgac actatggggc tagatggggc agaaggagca ggactgtgca tggatgacat 960

tccccacatc ttaaaaggtt gtatgccaga cagatatcag tttaattccc gtaaaccaat 1020

tacacctgag cattctactt ttatcacctc tccatctctg aaggacagga ttcactgtgt 1080

ggcttatgtc ttagacatca actctattga caatctctac tctaaaatgt tggcaaaagt 1140

gaagcaagtt cacaaagaag tattaaactg tggtatagca tatgtggcct tgcttactaa 1200

agtggatgat tgcagtgagg ttcttcaaga caacttttta aacatgagta gatctatgac 1260

ttctcaaagc cgggtcatga atgtccataa aatgctaggc attcctattt ccaatatttt 1320

gatggttgga aattatgctt cagatttgga actggacccc atgaaggata ttctcatcct 1380

ctctgcactg aggcagatgc tgcgggctgc agatgatttt ttagaagatt tgcctcttga 1440

ggaaactggt gcaattgaga gagcgttaca gccctgcatt tgagataagt tgccttgatt 1500

ctgacatttg gcccagcctg tactggtgtg ccgcaatgag agtcaatctc tattgacagc 1560

ctgcttcaga ttttgctttt gttcgttttg ccttctgtcc ttggaacagt catatctcaa 1620

gttcaaaggc caaaacctga gaagcggtgg gctaagatag gtcctactgc aaaccacccc 1680

tccatatttc cgtaccattt acaattcagt ttctgtgaca tctttttaaa ccactggagg 1740

aaaaatgaga tattctctaa tttattcttc tataacactc tatatagagc tatgtgagta 1800

ctaatcacat tgaataatag ttataaaatt attgtataga catctgcttc ttaaacagat 1860

tgtgagttct ttgagaaaca gcgtggattt tacttatctg tgtattcaca gagcttagca 1920

cagtgcctgg taatgagcaa gcatacttgc cattactttt ccttcccact ctctccaaca 1980

tcacattcac tttaaatttt tctgtatata gaaaggaaaa ctagcctggg caacatgatg 2040

aaaccccatc tccactgc 2058

<210> 6

<211> 2607

<212> DNA

<213> Homo sapiens

<220>

<221> transcription factor ISGF-3

<222> (1)..(2607)

<223> GenBank accession No. M97936

<400> 6

attaaacctc tcgccgagcc cctccgcaga ctctgcgccg gaaagtttca tttgctgtat 60

gccatcctcg agagctgtct aggttaacgt tcgcactctg tgtatataac ctcgacagtc 120

ttggcaccta acgtgctgtg cgtagctgct cctttggttg aatccccagg cccttgttgg 180

ggcacaaggt ggcaggatgt ctcagtggta cgaacttcag cagcttgact caaaattcct 240

ggagcaggtt caccagcttt atgatgacag ttttcccatg gaaatcagac agtacctggc 300

acagtggtta gaaaagcaag actgggagca cgctgccaat gatgtttcat ttgccaccat 360

ccgttttcat gacctcctgt cacagctgga tgatcaatat agtcgctttt ctttggagaa 420

taacttcttg ctacagcata acataaggaa aagcaagcgt aatcttcagg ataattttca 480

ggaagaccca atccagatgt ctatgatcat ttacagctgt ctgaaggaag aaaggaaaat 540

tctggaaaac gcccagagat ttaatcaggc tcagtcgggg aatattcaga gcacagtgat 600

gttagacaaa cagaaagagc ttgacagtaa agtcagaaat gtgaaggaca aggttatgtg 660

tatagagcat gaaatcaaga gcctggaaga tttacaagat gaatatgact tcaaatgcaa 720

aaccttgcag aacagagaac acgagaccaa tggtgtggca aagagtgatc agaaacaaga 780

acagctgtta ctcaagaaga tgtatttaat gcttgacaat aagagaaagg aagtagttca 840

caaaataata gagttgctga atgtcactga acttacccag aatgccctga ttaatgatga 900

actagtggag tggaagcgga gacagcagag cgcctgtatt ggggggccgc ccaatgcttg 960

cttggatcag ctgcagaact ggttcactat agttgcggag agtctgcagc aagttcggca 1020

gcagcttaaa aagttggagg aattggaaca gaaatacacc tacgaacatg accctatcac 1080

aaaaaacaaa caagtgttat gggaccgcac cttcagtctt ttccagcagc tcattcagag 1140

ctcgtttgtg gtggaaagac agccctgcat gccaacgcac cctcagaggc cgctggtctt 1200

gaagacaggg gtccagttca ctgtgaagtt gagactgttg gtgaaattgc aagagctgaa 1260

ttataatttg aaagtcaaag tcttatttga taaagatgtg aatgagagaa atacagtaaa 1320

aggatttagg aagttcaaca ttttgggcac gcacacaaaa gtgatgaaca tggaggagtc 1380

caccaatggc agtctggcgg ctgaatttcg gcacctgcaa ttgaaagaac agaaaaatgc 1440

tggcaccaga acgaatgagg gtcctctcat cgttactgaa gagcttcact cccttagttt 1500

tgaaacccaa ttgtgccagc ctggtttggt aattgacctc gagacgacct ctctgcccgt 1560

tgtggtgatc tccaacgtca gccagctccc gagcggttgg gcctccatcc tttggtacaa 1620

catgctggtg gcggaaccca ggaatctgtc cttcttcctg actccaccat gtgcacgatg 1680

ggctcagctt tcagaagtgc tgagttggca gttttcttct gtcaccaaaa gaggtctcaa 1740

tgtggaccag ctgaacatgt tgggagagaa gcttcttggt cctaacgcca gccccgatgg 1800

tctcattccg tggacgaggt tttgtaagga aaatataaat gataaaaatt ttcccttctg 1860

gctttggatt gaaagcatcc tagaactcat taaaaaacac ctgctccctc tctggaatga 1920

tgggtgcatc atgggcttca tcagcaagga gcgagagcgt gccctgttga aggaccagca 1980

gccggggacc ttcctgctgc ggttcagtga gagctcccgg gaaggggcca tcacattcac 2040

atgggtggag cggtcccaga acggaggcga acctgacttc catgcggttg aaccctacac 2100

gaagaaagaa ctttctgctg ttactttccc tgacatcatt cgcaattaca aagtcatggc 2160

tgctgagaat attcctgaga atcccctgaa gtatctgtat ccaaatattg acaaagacca 2220

tgcctttgga aagtattact ccaggccaaa ggaagcacca gagccaatgg aacttgatgg 2280

ccctaaagga actggatata tcaagactga gttgatttct gtgtctgaag tgtaagtgaa 2340

cacagaagag tgacatgttt acaaacctca agccagcctt gctcctggct ggggcctgtt 2400

gaagatgctt gtattttact tttccattgt aattgctatc gccatcacag ctgaacttgt 2460

tgagatcccc gtgttactgc ctatcagcat tttactactt taaaaaaaaa aaaaaaagcc 2520

aaaaaccaaa tttgtattta aggtatataa attttcccaa aactgatacc ctttgaaaaa 2580

gtataaataa aatgagcaaa agttgaa 2607

<210> 7

<211> 1584

<212> DNA

<213> Homo sapiens

<220>

<221> ISGF3-gamma

<222> (1)..(1584)

<223> GenBank accession No. M87503

<400> 7

gatcagaggg cgatcagctg gacagcaact caggatggca tcaggcaggg cacgctgcac 60

ccgaaaactc cggaactggg tggtggagca agtggagagt gggcagtttc ccggagtgtg 120

ctgggatgat acagctaaga ccatgttccg gattccctgg aaacatgcag gcaagcagga 180

cttccgggag gaccaggatg ctgccttctt caaggcctgg gcaatattta agggaaagta 240

taaggagggg gacacaggag gtccagctgt ctggaagact cgcctgcgct gtgcactcaa 300

caagagttct gaatttaagg aggttcctga gaggggccgc atggatgttg ctgagcccta 360

caaggtgtat cagttgctgc caccaggaat cgtctctggc cagccaggga ctcagaaagt 420

accatcaaag cgacagcaca gttctgtgtc ctctgagagg aaggaggaag aggatgccat 480

gcagaactgc acactcagtc cctctgtgct ccaggactcc ctcaataatg aggaggaggg 540

ggccagtggg ggagcagtcc attcagacat tgggagcagc agcagcagca gcagccctga 600

gccacaggaa gttacagaca caactgaggc cccctttcaa ggggatcaga ggtccctgga 660

gtttctgctt cctccagagc cagactactc actgctgctc accttcatct acaacgggcg 720

cgtggtgggc gaggcccagg tgcaaagcct ggattgccgc cttgtggctg agccctcagg 780

ctctgagagc agcatggagc aggtgctgtt ccccaagcct ggcccactgg agcccacgca 840

gcgcctgctg agccagcttg agaggggcat cctagtggcc agcaaccccc gaggcctctt 900

cgtgcagcgc ctttgcccca tccccatctc ctggaatgca ccccaggctc cacctgggcc 960

aggcccgcat ctgctgccca gcaacgagtg cgtggagctc ttcagaaccg cctacttctg 1020

cagagacttg gtcaggtact ttcagggcct gggcccccca ccgaagttcc aggtaacact 1080

gaatttctgg gaagagagcc atggctccag ccatactcca cagaatctta tcacagtgaa 1140

gatggagcag gcctttgccc gatacttgct ggagcagact ccagagcagc aggcagccat 1200

tctgtccctg gtgtagagcc tgggggaccc atcttccacc tcacctcttt gttcttcctg 1260

tctcctttga agtagactca ttcttcacac gattgacctg tcctctttgt gataattctc 1320

agtagttgtc cgtgataatc gtgtcctgaa aatcctcgca cacactggct ggtggagaac 1380

tcaaggctaa ttttttatcc tttttttttt tttatttttg agatatacgc cctctttcat 1440

ctgtaaggga ctaggaaatt ccaaatggtg tgaacccagg gggcctttcc ctcttccctg 1500

acctcccaac tctaaagcca agcactttat atttttctct tagatattca ctaaggactt 1560

aaaataaaat ttttttgaaa gagg 1584

<210> 8

<211> 483

<212> DNA

<213> Homo sapiens

<220>

<221> hepatitis C-associated microtubular aggregate

<222> (1)..(483)

<223> GenBank accession No. D28915

<400> 8

cctctggttg cctttcctga gataatccac taagaatatt ttgtgtttct tttctcaggg 60

aatctaaggg aggaaattat caactgtgca caaggaaaaa aatagatatg tgaaaggttc 120

acgtaaattt cctcacatca cagaagatta aaattcagaa aggagaaaac acagaccaaa 180

gagaagtatc taagaccaaa gggatgtgtt ttattaatgt ctaggatgaa gaaatgcata 240

gaacattgta gtacttgtaa ataactagaa ataacatgat ttagtcataa ttgtgaaaaa 300

taataataat ttttcttgga tttatgttct gtatctgtga aaaaataaat ttcttataaa 360

actcgggtct aacttgagag tgtgtgtgat tttggaaaaa ttatgatttg tcagcatctt 420

ctgatattca ctgctttcat cttaattttg ccttctgatt ttatttctaa agtatgtgat 480

ttt 483

<210> 9

<211> 1642

<212> DNA

<213> Homo sapiens

<220>

<221> interferon-inducible 56 Kd protein_(IFIT-1)

<222> (1)..(1642)

<223> GenBank accession No. M24594

<400> 9

ccagatctca gaggagcctg gctaagcaaa accctgcaga acggctgcct aatttacagc 60

aaccatgagt acaaatggtg atgatcatca ggtcaaggat agtctggagc aattgagatg 120

tcactttaca tgggagttat ccattgatga cgatgaaatg cctgatttag aaaacagagt 180

cttggatcag attgaattcc tagacaccaa atacagtgtg ggaatacaca acctactagc 240

ctatgtgaaa cacctgaaag gccagaatga ggaagccctg aagagcttaa aagaagctga 300

aaacttaatg caggaagaac atgacaacca agcaaatgtg aggagtctgg tgacctgggg 360

caactttgcc tggatgtatt accacatggg cagactggca gaagcccaga cttacctgga 420

caaggtggag aacatttgca agaagctttc aaatcccttc cgctatagaa tggagtgtcc 480

agaaatagac tgtgaggaag gatgggcctt gctgaagtgt ggaggaaaga attatgaacg 540

ggccaaggcc tgctttgaaa aggtgcttga agtggaccct gaaaaccctg aatccagcgc 600

tgggtatgcg atctctgcct atcgcctgga tggctttaaa ttagccacaa aaaatcacaa 660

gccattttct ttgcttcccc taaggcaggc tgtccgctta aatccagaca atggatatat 720

taaggttctc cttgccctga agcttcagga tgaaggacag gaagctgaag gagaaaagta 780

cattgaagaa gctctagcca acatgtcctc acagacctat gtctttcgat atgcagccaa 840

gttttaccga agaaaaggct ctgtggataa agctcttgag ttattaaaaa aggccttgca 900

ggaaacaccc acttctgtct tactgcatca ccagataggg ctttgctaca aggcacaaat 960

gatccaaatc aaggaggcta caaaagggca gcctagaggg cagaacagag aaaagctaga 1020

caaaatgata agatcagcca tatttcattt tgaatctgca gtggaaaaaa agcccacatt 1080

tgaggtggct catctagacc tggcaagaat gtatatagaa gcaggcaatc acagaaaagc 1140

tgaagagaat tttcaaaaat tgttatgcat gaaaccagtg gtagaagaaa caatgcaaga 1200

catacatttc tactatggtc ggtttcagga atttcaaaag aaatctgacg tcaatgcaat 1260

tatccattat ttaaaagcta taaaaataga acaggcatca ttaacaaggg ataaaagtat 1320

caattctttg aagaaattgg ttttaaggaa acttcggaga aaggcattag atctggaaag 1380

cttgagcctc cttgggttcg tctataaatt ggaaggaaat atgaatgaag ccctggagta 1440

ctatgagcgg gccctgagac tggctgctga ctttgagaac tctgtgagac aaggtcctta 1500

ggcacccaga tatcagccac tttcacattt catttcattt tatgctaaca tttactaatc 1560

atcttttctg cttactgttt tcagaaacat tataattcac tgtaatgatg taattcttga 1620

ataataaatc tgacaaaata tt 1642

<210> 10

<211> 853

<212> DNA

<213> Homo sapiens

<220>

<221> interferon-inducible protein 9-27

<222> (1)..(853)

<223> GenBank accession No. J04164

<400> 10

ctagtcctga cttcacttct gatgaggaag cctctctcct tagccttcag cctttcctcc 60

caccctgcca taagtaattt gatcctcaag aagttaaacc acacctcatt ggtccctggc 120

taattcacca atttacaaac agcaggaaat agaaacttaa gagaaataca cacttctgag 180

aaactgaaac gacaggggaa aggaggtctc actgagcacc gtcccagcat ccggacacca 240

cagcggccct tcgctccacg cagaaaacca cacttctcaa accttcactc aacacttcct 300

tccccaaagc cagaagatgc acaaggagga acatgaggtg gctgtgctgg gggcaccccc 360

cagcaccatc cttccaaggt ccaccgtgat caacatccac agcgagacct ccgtgcccga 420

ccatgtcgtc tggtccctgt tcaacaccct cttcttgaac tggtgctgtc tgggcttcat 480

agcattcgcc tactccgtga agtctaggga caggaagatg gttggcgacg tgaccggggc 540

ccaggcctat gcctccaccg ccaagtgcct gaacatctgg gccctgattc tgggcatcct 600

catgaccatt ggattcatcc tgtcactggt attcggctct gtgacagtct accatattat 660

gttacagata atacaggaaa aacggggtta ctagtagccg cccatagcct gcaacctttg 720

cactccactg tgcaatgctg gccctgcacg ctggggctgt tgcccctgcc cccttggtcc 780

tgcccctaga tacagcagtt tatacccaca cacctgtcta cagtgtcatt caataaagtg 840

cacgtgcttg tga 853

<210> 11

<211> 1377

<212> DNA

<213> Homo sapiens

<220>

<221> 2-5A synthetase induced by interferon

<222> (1)..(1377)

<223> X04371

<400> 11

aacgaaacca acagcagtcc aagctcagtc agcagaagag ataaaagcaa acaggtctgg 60

gaggcagttc tgttgccact ctctctcctg tcaatgatgg atctcagaaa taccccagcc 120

aaatctctgg acaagttcat tgaagactat ctcttgccag acacgtgttt ccgcatgcaa 180

atcaaccatg ccattgacat catctgtggg ttcctgaagg aaaggtgctt ccgaggtagc 240

tcctaccctg tgtgtgtgtc caaggtggta aagggtggct cctcaggcaa gggcaccacc 300

ctcagaggcc gatctgacgc tgacctggtt gtcttcctca gtcctctcac cacttttcag 360

gatcagttaa atcgccgggg agagttcatc caggaaatta ggagacagct ggaagcctgt 420

caaagagaga gagcattttc cgtgaagttt gaggtccagg ctccacgctg gggcaacccc 480

cgtgcgctca gcttcgtact gagttcgctc cagctcgggg agggggtgga gttcgatgtg 540

ctgcctgcct ttgatgccct gggtcagttg actggcagct ataaacctaa cccccaaatc 600

tatgtcaagc tcatcgagga gtgcaccgac ctgcagaaag agggcgagtt ctccacctgc 660

ttcacagaac tacagagaga cttcctgaag cagcgcccca ccaagctcaa gagcctcatc 720

cgcctagtca agcactggta ccaaaattgt aagaagaagc ttgggaagct gccacctcag 780

tatgccctgg agctcctgac ggtctatgct tgggagcgag ggagcatgaa aacacatttc 840

aacacagccc agggatttcg gacggtcttg gaattagtca taaactacca gcaactctgc 900

atctactgga caaagtatta tgactttaaa aaccccatta ttgaaaagta cctgagaagg 960

cagctcacga aacccacgcc tgtgatcctg gacccggcgg accctacagg aaacttgggt 1020

ggtggagacc caaagcgttg gaggcagctg gcacaagagg ctgaggcctg gctgaattac 1080

ccatgcttta agaattggga tgggtcccca gtgagctcct ggattctgct ggtgagacct 1140

cctgcttcct ccctgccatt catccctgcc cctctccatg aagcttgaga catatagctg 1200

gagaccattc tttccaaaga acttacctct tgccaaaggc catttatatt catatagtga 1260

caggctgtgc tccatatttt acagtcattt tggtcacaat cgagggtttc tggaattttc 1320

acatcccttg tccagaattc attcccctaa gagtaataat aaataatctc taacacc 1377

<<210> 12

<211> 5503

<212> DNA

<213> Homo sapiens

<220>

<221> Wilms' tumor gene

<222> (1)..(5503)

<223> GenBank accession No. X69950

<220>

<221> misc_feature

<222> (2041)..(2041)

<223> undefined nucleotide

<220>

<221> misc_feature

<222> (2083)..(2083)

<223> undefined nucleotide

<220>

<221> misc_feature

<222> (2186)..(2186)

<223> undefined nucleotide

<220>

<221> misc_feature

<222> (2187)..(2187)

<223> undefined nucleotide

<220>

<221> misc_feature

<222> (2488)..(2488)

<223>  undefined  nucleotide

<220>

<221>  misc_feature

<222>  (3386)..(3386)

<223>  undefined  nucleotide

<220>

<221>  misc_feature

<222>  (3718)..(3718)

<223>  undefined  nucleotide

<220>

<221>  misc_feature

<222>  (3720)..(3720)

<223>  undefined  nucleotide

<220>

<221>  misc_feature

<222>  (3938)..(3938)

<223>  undefined  nucleotide

<400>  12

ggtgcgctca ggactggaag caactctcag ttcatcaatt tcatgggcct acgtatctgc      60

tctggccacg gcctggcctc catccccact gtggcattaa tgcagaacaa attgagacat     120

atcctctata taattagttc cagaatttaa aaagatttac ggtataataa tagaggcagt     180

gccgcggaat cctttcactg aggactctcg gggagctcgg ggaccatctc gcggtggcat     240

cagaacagac gagtaacccc agcggctcca ggcactgcga actgggggag agggggaaat     300

tttgcaccgc attcggcagt acctaacggt tccgggctcc cagatagggt cagcgaggag 360

agttaggaca gtaagtgggg actggttaaa aaaaatcgac ttaattttga aatgatcaga 420

agaagctggg agtagttatc acaaagcccc tcccttgccc cccccttttt ttaattgaag 480

gaatgacgtt gaaattttca ccgccagcgc ggggctggga accatcctaa acacttgccc 540

tgcagccccg agtgggcaga ttccgaagag ttggagtcgg cggggagggg aggaacagct 600

gttgggctgt cttgcgcgtg gggctcgggg cgcgggcccc ggggagaggc gctagtcgaa 660

agcttgttcc tctcccgcga caggcagcag cgaggtcgag ccactcttta ttacggcctg 720

cgggccccgc gcgcagtgtg gctgtccccg cccttgaccg accgcagcgc tttggggtgt 780

ttattcagtt gtccgcggcc gctgggtgac tcccggaggc ggcccaggcg tgcgcactgg 840

tccctggggt tgcggctgta gcagcccagc tccgcgctct gtcagatgca gtggacagcg 900

ccggggtgaa agtaagggtt gagaaatcct cactgccctg ctcctaccca gcctcgattt 960

tttcatattg caataattat gcaccttcga ggccgggatg cctgcgcttt gccagtgtat 1020

caacgccaca tggttttcca ggggctgtcc tcgcctctgc atcccattag ctctgaagag 1080

gtagaggggt ggtgggtaaa acctccaact ggacgttgag agacccgggt ttcagctcgg 1140

cgttgccact aacttgtttc ctgtccttaa acagtagtag taacaaaaac tgtaaagttg 1200

attgagtcaa aggcccacac tgctctccta tctaatcctc agaacaactc tatgaggtgg 1260

gcgctagtat cccattatcc cgtttgacca gtaaggaaac tgaggctcag agagactgcg 1320

gaactcgtct agggtcacac agctgttggt tacctggcag agctgaggtt ccattctggg 1380

gccaccaacc ccttgctctt tacgtgcgct ccatagactg cctctccttc ggctgtctgg 1440

gcctcagttt ccttctctgt aaaacgagaa gtttaatctg cagctttctt tgccaatcgg 1500

tggttcagac caggaacctc tgctacagat ctcggcgctg acaggggtaa gaaggtgggt 1560

gaaagaaggg ggcgcccagt tttccacagc ctgcttttct ctgggacctc gcgaagggcg 1620

ggcctcgcga gctaaaggag gtacaggaga gcgcctatcg tccgcggcgg gtgaaggtgc 1680

tacctgcctt cgtgctaggc tgtgagtcct ggtgcttagc tcagggcgcc aaggccagtg 1740

tagctggcat gtcccccttg gaaaacctca ggtctcccgc agagaacgtt acccacaaca 1800

aagaagagga cagagaggca tggagcgccc tgcgactgca ggagtacgtc agttccccag 1860

cgctggctta gtgtcgcctg ggcttccggg catgtggatc cgttggggtc gtacggagac 1920

ttcctgtcgg gtccctgggg tcctccgact gcggctcctc agcttagcac tttcttcttg 1980

gccccgcagg ctgcagggaa ctcctcccac ctctttagtc ggagaagtcc aagtcgggcg 2040

agggggcacc ccggggttcg caccggtgct cttcccctcc ccncccccac aaggattctg 2100

agaaaataaa tggcagagga gagaggagtt ctacatttgc ttggctctcc tttcctccta 2160

tccaccccta catccctcac cccggnncaa aaacttattt ttgaaaaatg ttggcagaga 2220

tttacgtgtc tttgccttac ctgggtttca caaacacaac gactcacatt caagccagcc 2280

tcccttcaga taacctcctc tccccccgct aaaagtgcca aggatggtaa aagaagaaac 2340

aatctcaatc ttttcgtttg gaaatgaaag tccccggctt ttcataaagg gctcctcgcc 2400

cctcacagtt gagtcctagt taagaaaaac gacttccaag tagaaataat aggcggggag 2460

aaggaaggga gatacaggga tctggggngt tcttagggca actggcagtg aattttgtct 2520

cgagagtcct ttctccactc aaaaaaccaa acgcgcgagc cccgcgaaag gtttagggat 2580

agatcgtgtg ggagaggact gagcagagag cgtgggggca gtgtcttgta gaatctttct 2640

tttcttaata ataattttaa aagcttctga gtggagacga cgcaaagtca agcagcaaag 2700

gtggcctggg aggcaagcgg agggctcaag tgccgcatct ttaccctcag ggtctcctgc 2760

gcctacggga tgcgcattcc caagaagtgc gcccttcgag taagtcctgg gcccgcacac 2820

acttcgggtc cgcagccaga atttaatggc gacaacgttt atgcaatgca agctaaaaac 2880

caaagcgtaa aaaattacta tgtcatttat tgaaacgcca ttctttgtca aactgcaact 2940

actttgcttc acataagttt ggctggaaag cttgcagccc cagcccgggc cagccaggta 3000

caggaggccg gactgcaacc ggttgcttcc ctcccgtcgc gcctggccgt cccacgctgc 3060

gccgtcgctg ctgcctcctg gcgcccctgg gattttatac gcacctctga aacacgctcc 3120

gctccggccc ccggttcttc tccttgccta ggggttgttt cccaatagat actgactcct 3180

ttagaagatc caaaaaccaa accaaaacac cccctacccg ccccaaacac ctgctctggg 3240

gcgcgggggc tgccaaacag agactagacg aagggagtca gatttagcga agctcttcga 3300

gctcccaaag attcgaacac taactcgcgc ccgtgggccg atggaggttc tccctactcc 3360

actccttggt ccccttaact ggcttncgcc tcctggtcaa tcactgagca accagaatgg 3420

tatcctcgac cagggccaca ggcagtgctc ggcggagtgg ctccaggagt tacccgctcc 3480

ctgccgggct tcgtatccaa accctcccct tcacccctcc tccccaaact gggcgccagg 3540

atgctccggc cggaatatac gcaggctttg ggcgtttgcc caagggtttt cttccctcct 3600

aaactagccg ctgttttccc ggcttaaccg tagaagaatt agatattcct cactggaaag 3660

ggaaactaag tgctgctgac tccaatttta ggtaggcggc aaccgccttc cgcctggngn 3720

aaacctcacc aagtaaacaa ctactagccg atcgaaatac gcccggctta taactggtgc 3780

aactcccggc cacccaactg agggacgttc gctttcagtc ccgacctctg gaacccacaa 3840

agggccacct ctttccccag tgaccccaag atcatggcca ctcccctacc cgacagttct 3900

agaagcaaga gccagactca agggtgcaaa gcaagggnat acgcttcttt gaagcttgac 3960

tgagttcttt ctgcgctttc ctgaagttcc cgccctcttg gagcctacct gcccctccct 4020

ccaaaccact cttttagatt aacaacccca tctctactcc caccgcattc gaccctgccc 4080

ggactcactg cttacctgaa cgactctcca gtgagacgag gctcccacac tggcgaaggc 4140

caagaagggg aggtgggggg agggttgtgc cacaccggcc agctgagagc gcgtgttggg 4200

ttgaagagga gggtgtctcc gagagggacg ctccctcgga cccgccctca ccccagctgc 4260

gagggcgccc ccaaggagca gcgcgcgctg cctggccggg cttgggctgc tgagtgaatg 4320

gagcggccga gcctcctggc tcctcctctt ccccgcgccg ccggcccctc ttatttgagc 4380

tttgggaagc tgagggcagc caggcagctg gggtaaggag ttcaaggcag cgcccacacc 4440

cgggggctct ccgcaacccg accgcctgtc cgctccccca cttcccgccc tccctcccac 4500

ctactcattc acccacccac ccacccagag ccgggacggc agcccaggcg cccgggcccc 4560

gccgtctcct cgccgcgatc ctggacttcc tcttgctgca ggacccggct tccacgtgtg 4620

tcccggagcc ggcgtctcag cacacgctcc gctccgggcc tgggtgccta cagcagccag 4680

agcagcaggg agtccgggac ccgggcggca tctgggccaa gttaggcgcc gccgaggcca 4740

gcgctgaacg tctccagggc cggaggagcc gcggggcgtc cgggtctgag cctcagcaaa 4800

tgggctccga cgtgcgggac ctgaacgcgc tgctgcccgc cgtcccctcc ctgggtggcg 4860

gcggcggctg tgccctgcct gtgagcggcg cggcgcagtg ggcgccggtg ctggactttg 4920

cgcccccggg cgcttcggct tacgggtcgt tgggcggccc cgcgccgcca ccggctccgc 4980

cgccaccccc gccgccgccg cctcactcct tcatcaaaca ggagccgagc tggggcggcg 5040

cggagccgca cgaggagcag tgcctgagcg ccttcactgt ccacttttcc ggccagttca 5100

ctggcacagc cggagcctgt cgctacgggc ccttcggtcc tcctccgccc agccaggcgt 5160

catccggcca ggccaggatg tttcctaacg cgccctacct gcccagctgc ctcgagagcc 5220

agcccgctat tcgcaatcag ggtaagtagg ccggggagcg ccccctacgc gcggggcagt 5280

ggcgccaggg actctccgct ctaggacacc cccctctcct accccttttg accgcagctc 5340

ttacccagct gcttcccaag ggccgtgagg atagcggaag cggcggctgg ggaggaggcc 5400

ggagagtggg agtgcacgca ggcactggcc cccgacatcc tccaaagcca ggcagagcta 5460

ggagcctgac tgttcgcaag agccgggagg gcgtctgggg ccc 5503

<210> 13

<211> 1816

<212> DNA

<213> Homo sapiens

<220>

<221> interferon regulatory factor 7B_IRF-7

<222> (1)..(1816)

<223> GenBank accession No. U53831

<400> 13

ggcacccagg gtccggcctg cgccttcccg ccaggcctgg acactggttc aacacctgtg 60

acttcatgtg tgcgcgccgg ccacacctgc agtcacacct gtagccccct ctgccaagag 120

atccataccg aggcagcgtc ggtggctaca agccctcagt ccacacctgt ggacacctgt 180

gacacctggc cacacgacct gtggccgcgg cctggcgtct gctgcgacag gagcccttac 240

ctcccctgtt ataacacctg accgccacct aactgcccct gcagaaggag caatggcctt 300

ggctcctgag agggcagccc cacgcgtgct gttcggagag tggctccttg gagagatcag 360

cagcggctgc tatgaggggc tgcagtggct ggacgaggcc cgcacctgtt tccgcgtgcc 420

ctggaagcac ttcgcgcgca aggacctgag cgaggccgac gcgcgcatct tcaaggcctg 480

ggctgtggcc cgcggcaggt ggccgcctag cagcagggga ggtggcccgc cccccgaggc 540

tgagactgcg gagcgcgccg gctggaaaac caacttccgc tgcgcactgc gcagcacgcg 600

tcgcttcgtg atgctgcggg ataactcggg ggacccggcc gacccgcaca aggtgtacgc 660

gctcagccgg gagctgtgct ggcgagaagg cccaggcacg gaccagactg aggcagaggc 720

ccccgcagct gtcccaccac cacagggtgg gcccccaggg ccattcttgg cacacacaca 780

tgctggactc caagccccag gccccctccc tgccccagct ggtgacaagg gggacctcct 840

gctccaggca gtgcaacaga gctgcctggc agaccatctg ctgacagcgt catggggggc 900

agatccagtc ccaaccaagg ctcctggaga gggacaagaa gggcttcccc tgactggggc 960

ctgtgctgga ggcgaggccg cggccccaga gtccccgcac caggcagagc cgtacctgtc 1020

accctcccca agcgcctgca ccgcggtgca agagcccagc ccaggggcgc tggacgtgac 1080

catcatgtac aagggccgca cggtgctgca gaaggtggtg ggacacccga gctgcacgtt 1140

cctatacggc cccccagacc cagctgtccg ggccacagac ccccagcagg tagcattccc 1200

cagccctgcc gagctcccgg accagaagca gctgcgctac acggaggaac tgctgcggca 1260

cgtggcccct gggttgcacc tggagcttcg ggggccacag ctgtgggccc ggcgcatggg 1320

caagtgcaag gtgtactggg aggtgggcgg acccccaggc tccgccagcc cctccacccc 1380

agcctgcctg ctgcctcgga actgtgacac ccccatcttc gacttcagag tcttcttcca 1440

agagctggtg gaattccggg cacggcagcg ccgtggctcc ccacgctata ccatctacct 1500

gggcttcggg caggacctgt cagctgggag gcccaaggag aagagcctgg tcctggtgaa 1560

gctggaaccc tggctgtgcc gagtgcacct agagggcacg cagcgtgagg gtgtgtcttc 1620

cctggatagc agcagcctca gcctctgcct gtccagcgcc aacagcctct atgacgacat 1680

cgagtgcttc cttatggagc tggagcagcc cgcctagaac ccagtctaat gagaactcca 1740

gaaagctgga gcagcccacc tagagctggc cgcggccgcc cagtctaata aaaagaactc 1800

cagaacaaaa aaaaaa 1816

<210> 14

<211> 2056

<212> DNA

<213> Homo sapiens

<220>

<221> Interferon-induced 60 kDa protein (CIG49)

<222> (1)..(2056)

<223> GenBank accession No. AF026939

<400> 14

gtggaaacct cttcagcatt tgcttggaat cagtaagcta aaaacaaaat caaccgggac 60

cccagctttt cagaactgca gggaaacagc catcatgagt gaggtcacca agaattccct 120

ggagaaaatc ctcccacagc tgaaatgcca tttcacctgg aacttattca aggaagacag 180

tgtctcaagg gatctagaag atagagtgtg taaccagatt gaatttttaa acactgagtt 240

caaagctaca atgtacaact tgttggccta cataaaacac ctagatggta acaacgaggc 300

agccctggaa tgcttacggc aagctgaaga gttaatccag caagaacatg ctgaccaagc 360

agaaatcaga agtctagtca cttggggaaa ctacgcctgg gtctactatc acttgggcag 420

actctcagat gctcagattt atgtagataa ggtgaaacaa acctgcaaga aattttcaaa 480

tccatacagt attgagtatt ctgaacttga ctgtgaggaa gggtggacac aactgaagtg 540

tggaagaaat gaaagggcga aggtgtgttt tgagaaggct ctggaagaaa agcccaacaa 600

cccagaattc tcctctggac tggcaattgc gatgtaccat ctggataatc acccagagaa 660

acagttctct actgatgttt tgaagcaggc cattgagctg agtcctgata accaatacgt 720

caaggttctc ttgggcctga aactgcagaa gatgaataaa gaagctgaag gagagcagtt 780

tgttgaagaa gccttggaaa agtctccttg ccaaacagat gtcctccgca gtgcagccaa 840

attttacaga agaaaaggtg acctagacaa agctattgaa ctgtttcaac gggtgttgga 900

atccacacca aacaatggct acctctatca ccagattggg tgctgctaca aggcaaaagt 960

aagacaaatg cagaatacag gagaatctga agctagtgga aataaagaga tgattgaagc 1020

actaaagcaa tatgctatgg actattcgaa taaagctctt gagaagggac tgaatcctct 1080

gaatgcatac tccgatctcg ctgagttcct ggagacggaa tgttatcaga caccattcaa 1140

taaggaagtc cctgatgctg aaaagcaaca atcccatcag cgctactgca accttcagaa 1200

atataatggg aagtctgaag acactgctgt gcaacatggt ttagagggtt tgtccataag 1260

caaaaaatca actgacaagg aagagatcaa agaccaacca cagaatgtat ccgaaaatct 1320

gcttccacaa aatgcaccaa attattggta tcttcaagga ttaattcata agcagaatgg 1380

agatctgctg caagcagcca aatgttatga gaaggaactg ggccgcctgc taagggatgc 1440

cccttcaggc ataggcagta ttttcctgtc agcatctgag cttgaggatg gtagtgagga 1500

aatgggccag ggcgcagtca gctccagtcc cagagagctc ctctctaact cagagcaact 1560

gaactgagac agaggaggaa aacagagcat cagaagcctg cagtggtggt tgtgacgggt 1620

aggaggatag gaagacaggg ggccccaacc tgggattgct gagcagggaa gctttgcatg 1680

ttgctctaag gtacattttt aaagagttgt tttttggccg ggcgcagtgg ctcatgcctg 1740

taatcccagc actttgggag gccgaggtgg gcggatcacg aggtctggag tttgagacca 1800

tcctggctaa cacagtgaaa tcccgtctct actaaaaata caaaaaatta gccaggcgtg 1860

gtggctggca cctgtagtcc cagctacttg ggaggctgag gcaggagaat ggcgtgaacc 1920

tggaaggaag aggttgcagt gagccaagat tgcgcccctg cactccagcc tgggcaacag 1980

agcaagactc ggaattcctg cagcccgggg gatccactat tctagagcgc cgcaacggcc 2040

gtggagtcca gagatg 2056

<210> 15

<211> 1879

<212> DNA

<213> Homo sapiens

<220>

<221> nuclear autoantigen (SP-100)

<222> (1)..(1879)

<223> GenBank accession No. M60618

<400> 15

ctgaggccca cgcagggcct agggtgggaa gatggcaggt gggggcggcg acctgagcac 60

caggaggctg aatgaatgta tttcaccagt agcaaatgag atgaaccatc ttcctgcaca 120

cagccacgat ttgcaaagga tgttcacgga agaccagggt gtagatgaca ggctgctcta 180

tgacattgta ttcaagcact tcaaaagaaa taaggtggag atttcaaatg caataaaaaa 240

gacatttcca ttcctcgagg gcctccgtga tcgtgatctc atcacaaata aaatgtttga 300

agattctcaa gattcttgta gaaacctggt ccctgtacag agagtggtgt acaatgttct 360

tagtgaactg gagaagacat ttaacctgcc agttctggaa gcactgttca gcgatgtcaa 420

catgcaggaa taccccgatt taattcacat ttataaaggc tttgaaaatg taatccatga 480

caaattgcct ctccaagaaa gtgaagaaga agagagggag gagaggtctg gcctccaact 540

aagtcttgaa caaggaactg gtgaaaactc ttttcgaagc ctgacttggc caccttcggg 600

ttccccatct catgctggta caaccccacc tgaaaatgga ctctcagagc acccctgtga 660

aacagaacag ataaatgcaa agagaaaaga tacaaccagt gacaaagatg attcgctagg 720

aagccaacaa acaaatgaac aatgtgctca aaaggctgag ccaacagagt cctgcgaaca 780

aattgctgtc caagtgaata atggggatgc tggaagggag atgccctgcc cgttgccctg 840

tgatgaagaa agcccagagg cagagctaca caaccatgga atccaaatta attcctgttc 900

tgtgcgactg gtggatataa aaaaggaaaa gccattttct aattcaaaag ttgagtgcca 960

agcccaagca agaactcatc ataaccaggc atctgacata atagtcatca gcagtgagga 1020

ctctgaagga tccactgacg ttgatgagcc cttagaagtc ttcatctcag caccgagaag 1080

tgagcctgtg atcaataatg acaacccttt agaatcaaat gatgaaaagg agggccaaga 1140

agccacttgc tcacgacccc agattgtacc agagcccatg gatttcagaa aattatctac 1200

attcagagaa agttttaaga aaagagtgat aggacaagac cacgactttt cagaatccag 1260

tgaggaggag gcgcccgcag aagcctcaag cggggcactg agaagcaagc atggtgagaa 1320

ggctcctatg acttctagaa gtacatctac ttggagaata cccagcagga agagacgttt 1380

cagcagtagt gacttttcag acctgagtaa tggagaagag cttcaggaaa cctgcagctc 1440

atccctaaga agagggtcag gtaaagaaga ttaggatgcc aagacttggc ctgcagaatg 1500

tcaggaatgt gaattaaaag ctgctgtttc cagacgcttt ttattctgag caccttcact 1560

accttgtatc cagttcatct gggaactcct ttttgcattt tagaaaatgg aaagaggcag 1620

gaaattatga taaactcatg tttaacagaa agagtttcac tgactaaatg tatgtaatta 1680

tattttgttg ttgtagaaga aataaatagc aaatttgtgg tattcttttt tttaaacctg 1740

ctctcattcc tattaacact aagatcttag atttttatag tgataaatgg gttgacatca 1800

ttgtcgtttg taattgtaaa gcctcaaaag acaactgttc ctactatgta attatagaca 1860

gaaataaaaa cttcagatc 1879

<210> 16

<211> 3997

<212>  DNA

<213>  Homo sapiens

<220>

<221>  KIAA0793

<222>  (1)..(3997)

<223>  GenBank accession No. AB018336

<400>  16

ggcgacgccg acgcgagtct ggcggctgct gcttgcgact gcggaggccg ggcgaggccg      60

gtgaggacgc ggcgggcgag cgagaggccg aggtgttttc ttcactcatg gtgaagaatg     120

ggggagatag aaggaacata cagagtcctg cagactgcag ggatgcgctt gggtgcccag     180

acccctgtgg gagttagcac ccttgagcct gggcagactc tcttgcccag aatgcaagag     240

aagcacctgc acctcagagt aaagctgctg gacaacacca tggaaatatt tgacattgag     300

cctaaatgcg atggccaggt attactgaca caagtgtgga agcgtttaaa cctggtagaa     360

tgtgactact tcgggatgga gtttcaaaat actcagtcct actggatttg gcttgaacct     420

atgaaaccca tcattaggca aatacgaagg ccaaagaatg tggtgcttcg cctagctgta     480

aaattttttc cacctgatcc tggtcagcta caagaagaat atacaagata cttgtttgcc     540

ttgcaactta agagagacct gctggaagag cgtttgacct gtgctgacac cacagcggcc     600

cttctcacgt cccatctcct gcagtcggaa ataggagatt acgatgaaac gctggaccga     660

gagcacctca aagtgaacga gtatttgcct ggccagcagc actgccttga gaagatacta     720

gaattccatc agaagcacgt gggccagaca cctgctgagt cggatttcca ggtgctcgaa     780

attgctcgaa agttggaaat gtacggcatc agatttcaca tggcttctga cagggaagga     840

accaagattc aactggcagt ttcccacatg ggtgtactcg tgttccaggg caccaccaaa     900

atcaacactt tcaactggtc caaggtccgt aaactaagct tcaagaggaa aagatttctt     960

atcaaacttc atccagaggt tcatggacct taccaggaca cattagaatt tttgttgggt    1020

agtagagatg aatgtaagaa cttctggaag atttgtgtgg agtatcacac cttttttaga    1080

cttttggacc aacctaagcc aaaagcaaaa gccgtcttct tcagccgggg ctcctccttc    1140

agatacagtg gaagaactca gaaacaacta gtagattatt tcaaagacag tggaatgaag    1200

agaattccat atgaaagaag gcacagcaag acccacacgt ccgttcgagc tctgactgca 1260

gacctaccaa aacagagcat ctcattcccc gagggattga ggactcctgc ctccccatct 1320

tcagcgaatg ccttttactc gctctctccc tccactctgg tcccctctgg cctgccagag 1380

tttaaggaca gcagcagctc cctcacagat ccccaggttt cctacgtcaa gagtccagct 1440

gcagagaggc gcagtggagc agtggctgga ggccccgaca caccatcggc ccagcccctc 1500

gggccccccg cactccagcc tggtccaggc ctttccacga agagtcctca gccttctccc 1560

tccagccgga agagccccct gagtctgagc cctgcatttc aggtgccttt gggcccagct 1620

gaacagggct catccccact cctgagccct gtcctcagtg atgctggcgg agccgggatg 1680

gactgcgagg agcccagaca caagcgcgtg cctgcagacg aggcctactt catagtcaaa 1740

gagattctcg ctacagaacg aacatacctc aaggatttag aagttattac cgtgtggttc 1800

cgcagcgcag tggtgaagga ggacgccatg cctgcgactc tgatgacgct gctcttctcc 1860

aacatcgatc ccatctatga gttccacaga ggcttcctgc gcgaggtgga gcagaggctg 1920

gcactctggg aagggccctc caaagcccac acaaaaggca gtcatcaacg aatcggggac 1980

atcctgctca ggaacatgcg ccagttaaag gagtttacca gctacttcca aagacatgac 2040

gaggtcctaa cagaactgga aaaggctacc aaacgctgta agaagttgga ggcagtgtac 2100

aaggagtttg agctgcagaa ggtctgctac ttgcctctca acacgttcct gctgaagccc 2160

atccagcggc tgctgcacta ccgcctgctg ctgcgccgcc tatgcggaca ttacagcccc 2220

gggcaccatg actacgctga ctgccatgac gccctgaaag ccatcacaga ggtgaccacc 2280

acactacagc acattctcat ccggctggag aacctgcaga agctaacgga gctgcagcgg 2340

gacctggtgg gcatagagaa cctcattgct cctggcaggg agttcatccg tgagggctgc 2400

cttcacaagc tcaccaagaa gggcctgcag cagaggatgt tttttctgtt ctcagatatg 2460

ttgctgtaca caagcaaagg agttgcaggg accagccact tccggatccg gggcctcctt 2520

cccctccaag gcatgctggt ggaagaaagt gataacgagt ggtctgttcc acactgtttc 2580

accatctacg cggctcagaa aacaatcgtg gtggcagcca gcactcggct ggagaaagag 2640

aagtggatgc tggacctgaa ctccgcgatc caagcagcca agagtggcgg tgacacggcc 2700

cctgcactgc caggccgcac tgtgtgcact cgtcccccca gatcccccaa cgaggtatct 2760

ctggagcagg agtcagaaga tgatgctcgg ggtgtccgca gctccctgga ggggcatggc 2820

cagcaccggg ccaacaccac aatgcacgtg tgctggtacc ggaacaccag cgtgtccagg 2880

gcagaccaca gtgcagctgt cgagaaccag ctttcaggat atctgctaag aaagttcaaa 2940

aacagtcatg gctggcagaa gctctgggtc gtctttacca acttctgttt gttcttctac 3000

aaaactcatc aggatgacta cccactggcc agcctcccgc tgctgggcta cagcgtgagc 3060

atccccaggg aggccgatgg catacacaaa gactatgttt tcaagctcca gttcaaatcc 3120

cacgtctact tcttccgggc tgagagcaag tacacatttg aaaggtggat ggaggtgatc 3180

cagggggcca gcagctcagc cgggagggcc ccaagcatcg tgcaggatgg cccccaaccc 3240

tcctcagggc tggaggggat ggtcaggggg aaggaggaat gacgctcaac ctgcccaggt 3300

ttggacacaa ctacaaagaa cagcaggaca cagaggtgac ctctgtcctg aggcttctca 3360

acagatggga agtggctgtg gtctcactgg atccccactg gcaccagcag tgtgggtggg 3420

cctcatgtaa catctgggag gggcttcatc cccccaccca ggacctagtg catgccagca 3480

gctatctggg gccctgggaa aaatgtgcga gtcttgagcg cggagccgct caagccacag 3540

ctcccaggcc cctggctcaa agacgcagac aaggcctgag cagtgctctc ggcatcggac 3600

caaagcctgg gcacaccctg cctctctccc cagagcaggg tccctgccga ggactggcct 3660

agagcaagca ctggaaaaga ggccctgcca tacaccctgc gtacccactg ccaggaccct 3720

ctcagacaag cgtggcacag ccatgctgac cttccatctg gtgaaccaag tggcagcccc 3780

aggggcctgc cctgcaggtc acagctaaac aagtctggca gaagccacgc ttgttcccca 3840

tgtacctcta gagaagcaga aaccaaagtc cccctgtgcc ctgggagggt ggggccgtct 3900

aatttattac tgcccagcat tccttccaac gggaagtaga tgggcgactg ctttgttcac 3960

acacatttga ttaaaaataa acaaacagca tctcccc 3997

<210> 17

<211> 2905

<212> DNA

<213> Homo sapiens

<220>

<221> 2'5' oligoadenylate synthetase (p69 2-5A synthetase)

<222> (1)..(2905)

<223>  GenBank accession No. M87434

<400>  17

cggcagccag ctgagagcaa tgggaaatgg ggagtcccag ctgtcctcgg tgcctgctca      60

gaagctgggt tggtttatcc aggaatacct gaagccctac gaagaatgtc agacactgat     120

cgacgagatg gtgaacacca tctgtgacgt ctgcaggaac cccgaacagt tccccctggt     180

gcagggagtg gccataggtg gctcctatgg acggaaaaca gtcttaagag gcaactccga     240

tggtaccctt gtccttttct tcagtgactt aaaacaattc caggatcaga agagaagcca     300

acgtgacatc ctcgataaaa ctggggataa gctgaagttc tgtctgttca cgaagtggtt     360

gaaaaacaat ttcgagatcc agaagtccct tgatgggtcc accatccagg tgttcacaaa     420

aaatcagaga atctctttcg aggtgctggc cgccttcaac gctctgagct taaatgataa     480

tcccagcccc tggatctatc gagagctcaa aagatccttg gataagacaa atgccagtcc     540

tggtgagttt gcagtctgct tcactgaact ccagcagaag ttttttgaca accgtcctgg     600

aaaactaaag gatttgatcc tcttgataaa gcactggcat caacagtgcc agaaaaaaat     660

caaggattta ccctcgctgt ctccgtatgc cctggagctg cttacggtgt atgcctggga     720

acaggggtgc agaaaagaca actttgacat tgctgaaggc gtcagaacgg ttctggagct     780

gatcaaatgc caggagaagc tgtgtatcta ttggatggtc aactacaact ttgaagatga     840

gaccatcagg aacatcctgc tgcaccagct ccaatcagcg aggccagtaa tcttggatcc     900

agttgaccca accaataatg tgagtggaga taaaatatgc tggcaatggc tgaaaaaaga     960

agctcaaacc tggttgactt ctcccaacct ggataatgag ttacctgcac catcttggaa    1020

tgtcctgcct gcaccactct tcacgacccc aggccacctt ctggataagt tcatcaagga    1080

gtttctccag cccaacaaat gcttcctaga gcagattgac agtgctgtta acatcatccg    1140

tacattcctt aaagaaaact gcttccgaca atcaacagcc aagatccaga ttgtccgggg    1200

aggatcaacc gccaaaggca cagctctgaa gactggctct gatgccgatc tcgtcgtgtt    1260

ccataactca cttaaaagct acacctccca aaaaaacgag cggcacaaaa tcgtcaagga    1320

aatccatgaa cagctgaaag ccttttggag ggagaaggag gaggagcttg aagtcagctt    1380

tgagcctccc aagtggaagg ctcccagggt gctgagcttc tctctgaaat ccaaagtcct    1440

caacgaaagt gtcagctttg atgtgcttcc tgcctttaat gcactgggtc agctgagttc    1500

tggctccaca cccagccccg aggtttatgc agggctcatt gatctgtata aatcctcgga 1560

cctcccggga ggagagtttt ctacctgttt cacagtcctg cagcgaaact tcattcgctc 1620

ccggcccacc aaactaaagg atttaattcg cctggtgaag cactggtaca aagagtgtga 1680

aaggaaactg aagccaaagg ggtctttgcc cccaaagtat gccttggagc tgctcaccat 1740

ctatgcctgg gagcagggga gtggagtgcc ggattttgac actgcagaag gtttccggac 1800

agtcctggag ctggtcacac aatatcagca gctcggcatc ttctggaagg tcaattacaa 1860

ctttgaagat gagaccgtga ggaagtttct actgagccag ttgcagaaaa ccaggcctgt 1920

gatcttggac ccaggcgaac ccacaggtga cgtgggtgga ggggaccgtt ggtgttggca 1980

tcttctggac aaagaagcaa aggttaggtt atcctctccc tgcttcaagg atgggactgg 2040

aaacccaata ccaccttgga aagtgccgac aatgcagaca ccaggaagtt gtggagctag 2100

gatccatcct attgtcaatg agatgttctc atccagaagc catagaatcc tgaataataa 2160

ttctaaaaga aacttctgga gatcatctgg caatcgcttt taaagactcg gctcaccgtg 2220

agaaagagtc actcacatcc attcttccct tgatggtccc tattcctcct tcccttgcct 2280

tcttggactt cttgaaatca atcaagactg caaacccttt cataaagctg ccttgctgaa 2340

ctcctctctg caggagccct gcttaaaata gttgatgtca tcactttatg tgcatcttat 2400

ttctgtcaac ttgtattttt ttttcttgta tttttccaat tagctcctcc tttttccttc 2460

cagtctaaaa aaggaatcct ctgtgtcttc aaagcaaagc tctttacttt ccccttggtt 2520

ctcataactc tgtgatcttg ctctcggtgc ttccaactca tccacgtcct gtctgtttcc 2580

tctgtataca aaaccctttc tgcccctgct gacacagaca tcctctatgc cagcagccag 2640

gccaaccctt tcattagaac ttcaagctct ccaaaggctc agattataac tgttgtcata 2700

tttatatgag gctgttgtct tttccttctg agcctgcctt tatcccccca cccaggagta 2760

tcctcttgcc aaagcaaaag actttttcct tggctttagc cttaaagata cttgaaggtc 2820

taggtgcttt aacctcacat accctcactt aaacttttat cactgttgca tataccagtt 2880

gtgatacaat aaagaatgta tctgg 2905

<210> 18

<211> 840

<212>  DNA

<213>  Homo sapiens

<220>

<221>  histone H2A

<222>  (1)..(840)

<223>  GenBank accession No. Z80776

<400>  18

ggatgaattg tctctgtgtg ccctgtggta tgcaaattag gtgattctag cgtttctttg      60

caattggttg gttgttctag ccaatcaaat agcgttattt acaattcgac ctccaagtat     120

aatagtttct cacttgctaa caaaactcac ttttacattt ttgtcttcat tgcttaacat     180

cgtttttaag aattcaaaat gtccggacgc ggcaagcaag gcggaaaggc ccgagctaag     240

gctaagaccc gctcttcgcg ggccggactc cagttccctg tgggccgcgt acaccgcttg     300

ctccgcaagg gcaactactc cgagcgagtc ggggccggcg cgccagtgta tctggcggcg     360

gtgttggagt acctgaccgc cgagatcctg gagctggcgg gcaacgccgc ccgcgacaac     420

aagaagaccc gcatcatccc ccgacacctg cagctggcca tccgcaacga cgaggagcta     480

aacaagttgc tgggtaaagt cacaattgct cagggcggtg ttctgcccaa catccaggct     540

gtactgctcc ccaagaagac tgagagtcac cacaaggcca agggcaagta aaacgagaac     600

tcttatattg gctttttaag gaagcagtct taacaaaggc tcttttcaga gccacccatg     660

tattccttaa aagggctcac attttctgtg taaatagcta ttttgcagtt ttctttaata     720

atttttccat ttataagtgg gtgttcttaa cactgaatcg tgaaactaac cgtccatttt     780

aaagatgcca taaaaataca taagcaaatt ggcggggcgc ggttgcttac gcctgtaatc     840

<210>  19

<211>  1230

<212>  DNA

<213>  Homo sapiens

<220>

<221> GPR12 G protein coupled-receptor

<222> (1)..(1230)

<223> GenBank accession No. U18548

<400> 19

aagcttgtgg catttggtac tggtatctga gcaggggctg gctttctgtt tgtctgtgtg 60

ttttttgcat gatcttggat tgtcaccctg ctgtatttaa acattaaaaa gcctgtcttt 120

tcgttgaaga ggacaggggt taaaatgaat gaagacctga aggtcaattt aagcgggctg 180

cctcgggatt atttagatgc cgctgctgcg gagaacatct cggctgctgt ctcctcccgg 240

gttcctgccg tagagccaga gcctgagctc gtagtcaacc cctgggacat tgtcttgtgt 300

acctcgggaa ccctcatctc ctgtgaaaat gccattgtgg tccttatcat cttccacaac 360

cccagcctgc gagcacccat gttcctgcta ataggcagcc tggctcttgc agacctgctg 420

gccggcattg gactcatcac caattttgtt tttgcctacc tgcttcagtc agaagccacc 480

aagctggtca cgatcggcct cattgtcgcc tctttctctg cctctgtctg cagcttgctg 540

gctatcactg ttgaccgcta cctctcactg tactacgctc tgacgtacca ttcggagagg 600

acggtcacgt ttacctatgt catgctcgtc atgctctggg ggacctccat ctgcctgggg 660

ctgctgcccg tcatgggctg gaactgcctc cgagacgagt ccacctgcag cgtggtcaga 720

ccgctcacca agaacaacgc ggccatcctc tcggtgtcct tcctcttcat gtttgcgctc 780

atgcttcagc tctacatcca gatctgtaag attgtgatga ggcacgccca tcagatagcc 840

ctgcagcacc acttcctggc cacgtcgcac tatgtgacca cccggaaagg ggtctccacc 900

ctggctatca tcctggggac gtttgctgct tgctggatgc ctttcaccct ctattccttg 960

atagcggatt acacctaccc ctccatctat acctacgcca ccctcctgcc cgccacctac 1020

aattccatca tcaaccctgt catatatgct ttcagaaacc aagagatcca gaaagcgctc 1080

tgtctcattt gctgcggctg catcccgtcc agtctcgccc agagagcgcg ctcgcccagt 1140

gatgtgtagc acccttgcac ccaggaggac tctgcattta ccaagcactt ccactgcctg 1200

gccaaggttt gagatgcttc ccttgaattc 1230

<210> 20

<211> 5141

<212> DNA

<213> Homo sapiens

<220>

<221> steroid 21-hydroxylase (CYP21)

<222> (1)..(5141)

<223> GenBank accession No. M12792

<400> 20

agatctttct cccagtagct gctcagcatg gtggtggcat aagcccattt tccggagcca 60

gggattcagt tgcagcaaga catggcccgg tctgggaggt caaccatgaa gaaggcagta 120

gctgtcattg cccaacccca gaaatcccaa tcctgttttc tccctctcag tcctgatcat 180

ggattcagca gcagcgaact cgccaatgta gtgggtggca cagccagggt cttgactctg 240

gctctgcagt agcacagtct ggaaaagctc tgaggggaga gagaccccca ctggtccgag 300

ggtctggcac agagccagaa atggggggga aggtatgagg ctgggtcgcc tctgacctct 360

caggtaccat ccaggaggcc ctggcctctc actgaacccg gccactcctc tttggcatgg 420

cctcttccca aatccccaaa ctgcctcctt acccacaaaa gtggtctctg agtgtcagtc 480

cagtgggacc cccacccctt atggcttcag ttccccaaat agggctggac ccttgatcct 540

gatccagctg tggctatcca gccccttcct ggggactttg gactttgagg ggggcatgcc 600

acttgtgctg ggaatccata ctttccctgg ctggagtaga acctgtggac tgtagtcctg 660

agggcagtca tgttctgcct gtgcctggaa agacaagaaa cttgactgca gagagaagaa 720

agaggagaga ggaacagagc gaggaaaccg cccgtctccg ggcttttttc tgttccctat 780

ccttgacttt ctaagaccag tggggtcccc tcctctgctt ctttttcctg agttctgtga 840

aattccccaa ttcttatttt ttatctcaaa ccagctcaag gtgggctgtt ttcctttcaa 900

ccaaagaaag gtgctcctgg tggctaaagg tacatattcg acagctagat ttccaggctg 960

gaatcctgcc ctccacaaca tgcgaacaat acccgtgttg catatagagc atggctgtga 1020

agagttgagt gagtgcccac aaagcactta gagcagtgtc tggtacatgc tattactccg 1080

cagcgggaaa ccacttcctc ctttgtcttc tgggcacttt tgtgagtgaa aggaggcact 1140

aataacaatc acactgggat acctgtatat actggaatgc cccaggcaaa ccaggcttaa 1200

actgtattac tctatctgta gcttaaacta acaaacaacc cacacaaatc acattttgtt 1260

cttcaggcga ttcaggaagg cctattaggc agggactgcc attttctctc tgagacaaac 1320

atcattccat aaactggccc acggtgggtg gcagagggag agggcccagg tgggggcgga 1380

cactattgcc tgcacagtga tgtggaacca gaaagctgac tctggatgca ggaaaaaggt 1440

cagggttgca tttcccttcc ttgcttcttg atgggtgatc aatttttttg aaatacggac 1500

gtcccaaggc caatgagact ggtgtcattc cagaaaaggg ccactctgtg ggcgggtcgg 1560

tgggagggta cctgaaggtg gggtcaaggg aggccccaaa acagtctaca cagcaggagg 1620

gatggctggg gctcttgagc tataagtggc acctcagggc cctgacgggc gtctcgccat 1680

gctgctcctg ggcctgctgc tgctgcccct gctggctggc gcccgcctgc tgtggaactg 1740

gtggaagctc cggagcctcc acctcccgcc tcttgccccg ggcttcttgc acttgctgca 1800

gcccgacctc ccaatctatc tgcttggcct gactcagaaa ttcgggccca tctacaggct 1860

ccaccttggg ctgcaaggtg agaggctgat ctcgctctgg ccctcaccat aggagggggc 1920

ggaggtgacg gagagggtcc tctctccgct gacgctgctt tggctgtctc ccagatgtgg 1980

tggtgctgaa ctccaagagg accattgagg aagccatggt caaaaagtgg gcagactttg 2040

ctggcagacc tgagccactt acctgtaagg gctgggggca tttttctttt cttaaacaaa 2100

ttttttttta agagatgggt tcttgctatg ttgcccaggc tggtcttaaa ttcctagtct 2160

caaatgatcc tcccacctca gcctcaagtg tgagccacct ttggggcatc cccaatccag 2220

gtccctggaa gctcttgggg ggcatatctg gtggggagaa agcaggggtt ggggaggccg 2280

aagaaggtca ggccctcagc tgccttcatc agttcccacc ctccagcccc cacctcctcc 2340

tgcagacaag ctggtgtcta agaactaccc ggacctgtcc ttgggagact actccctgct 2400

ctggaaagcc cacaagaagc tcacccgctc agccctgctg ctgggcatcc gtgactccat 2460

ggagccagtg gtggagcagc tgacccagga gttctgtgag gtaaggctgg gctcctgagg 2520

ccacctcggg tcagcctcgc ctctcacagt agcccccgcc ctgccgctgc acagcggcct 2580

gctgaactca cactgtttct ccacagcgca tgagagccca gcccggcacc cctgtggcca 2640

ttgaggagga attctctctc ctcacctgca gcatcatctg ttacctcacc ttcggagaca 2700

agatcaaggt gcctcacagc ccctcaggcc cacccccagc ccctccctga gcctctcctt 2760

gtcctgaact gaaagtactc cctccttttc tggcaggacg acaacttaat gcctgcctat 2820

tacaaatgta tccaggaggt gttaaaaacc tggagccact ggtccatcca aattgtggac 2880

gtgattccct ttctcagggt gaggacctgg agcctagaca cccctgggtt gtaggggaga 2940

ggctggggtg gagggagagg ctccttccca cagctgcatt ctcatgcttc ctgccgcagt 3000

tcttccccaa tccaggtctc cggaggctga agcaggccat agagaagagg gatcacatcg 3060

tggagatgca gctgaggcag cacaaggtgg ggactgtacg tggacggcct cccctcggcc 3120

cacagccagt gatgctaccg gcctcagcat tgctatgagg cgggttcttt tgcatacccc 3180

agttatgggc ctgttgccac tctgtactcc tctccccagg ccagccgctc agcccgctcc 3240

tttcaccctc tgcaggagag cctcgtggca ggccagtgga gggacatgat ggactacatg 3300

ctccaagggg tggcgcagcc gagcatggaa gagggctctg gacagctcct ggaagggcac 3360

gtgcacatgg ctgcagtgga cctcctgatc ggtggcactg agaccacagc aaacaccctc 3420

tcctgggccg tggttttttt gcttcaccac cctgaggtgc gtcctgggga caagcaaaag 3480

gctccttccc agcaacctgg ccagggcggt gggcaccctc actcagctct gagcactgtg 3540

cggctggggc tgtgcttgcc tcaccggcac tcaggctcac tgggttgctg agggagcggc 3600

tggaggctgg gcagctgtgg gctgctgggg caggactcca cccgatcatt ccccagattc 3660

agcagcgact gcaggaggag ctagaccacg aactgggccc tggtgcctcc agctcccggg 3720

tcccctacaa ggaccgtgca cggctgccct tgctcaatgc caccatcgcc gaggtgctgc 3780

gcctgcggcc cgttgtgccc ttagccttgc cccaccgcac cacacggccc agcaggtgac 3840

tcccgagggt tggggatgag tgaggaaagc ccgagcccag ggaggtcctg gccagcctct 3900

aactccagcc cccttcagca tctccggcta cgacatccct gagggcacag tcatcattcc 3960

gaacctccaa ggcgcccacc tggatgagac ggtctgggag aggccacatg agttctggcc 4020

tggtatgtgg gggccggggg cctgccgtca aaatgtggtg gaggctggtc cccgctgccg 4080

ctgaacgcct ccccacccac ctgtccaccc gcccgcagat cgcttcctgg agccaggcaa 4140

gaactccaga gctctggcct tcggctgcgg tgccccggtg tgcctgggcg agccgctggc 4200

gcgcctggac ctcttcgtgg tgctgacccg actgctgcag gccttcacgc tgctgccctc 4260

cggggacgcc ctgccctccc tgcagcccct gccccactgc agtgtcatcc tcaagatgca 4320

gcctttccaa gtgcggctgc agccccgggg gatgggggcc cacagcccag gccagaacca 4380

gtgatggggc aggaccgatg ccagccgggt acctcagttt ctcctttatt gctcccgtac 4440

gaacccctcc cctcccccct gtaaacacag tgctgcgaga tcgctggcag agaaggcttc 4500

ctccagcggc tgggtggtga aggaccctgg ctcttctctc ggggcgaccc ctcagtgctc 4560

ggcagtcata ctggggtgcg agagaggtgg gcagcagctc agcctccccc cgctggggag 4620

cgaaagtttc ttggtctcag cttcatttcc gtgaagggca ccgagaactc gaagcccttc 4680

cagtggtacc agctcactcc ctgggaaagg ggttgtcaag agagagtcaa agccggatgt 4740

cccatctgct cttcccgttc cccttaagga ggtagctccc agcactcaac caacctcccc 4800

gcagagctcc cttcctgacc ctccgctgca gaggattgag gcttaattct gagctggccc 4860

tttccagcca ataaatcaac tccagctccc tctgcgaggc tggcatgatt gttccatttc 4920

acccagccgc tcagtccctt gcctgttaca ctgtggggct gaaacctagg caggccgagc 4980

cccagccacc ccagctctga gccggcctcc ccacccctca cctgatggtc cactgtgctc 5040

ccgtagagcc cgttgaggtt ggcgtagtgg cagttcctgt accaccaggc ccctcggtag 5100

gagacagcgc aggagatgag caagctgttg gggtcccgat c 5141

<210> 21

<211> 1117

<212> DNA

<213> Homo sapiens

<220>

<221> putative tumor suppressor protein (101F6)

<222> (1)..(1117)

<223> GenBank accession No. AF040704

<400> 21

tccggaagta aagcggctcc gtgacggagc ggcggtgcgc gcggcagggc ccggagtatc 60

ccgctttctt tggaggaaac caccgcatca gatctgcgct gcggcagagg caggctacaa 120

ccactagcac ggctgacgat ggccctttct gcggagaccg agtcacacat ctaccgagct 180

ctgcgtactg cttctggcgc tgccgcccac cttgtggccc tgggctttac catctttgtg 240

gctgtgcttg ccaggcctgg ctccagcctg ttctcctggc acccggtgct tatgtctttg 300

gctttctcct tcctgatgac cgaggcacta ctggtgtttt ctcctgagag ttcgctgctg 360

cactccctct cacggaaagg ccgagcacgc tgccactggg tgctgcagct gctggccctg 420

ctgtgtgcac tgctgggcct cggccttgtc atcctccaca aagagcagct tggcaaagcc 480

cacctggtta cgcggcatgg gcaggcaggg ctgctggctg tgctgtgggc agggctgcag 540

tgctcaggtg gggtggggct gctctacccc aagctgctgc cccgatggcc cctggcgaag 600

ctcaagctat accatgctac ttctgggctg gtgggctacc tgctgggtag tgccagcctc 660

ttgctgggca tgtgctcact ctggttcact gcctctgtca ctggtgcagc ctggtacctg 720

gctgtattat gccctgtcct caccagcttg gtcattatga accaggtgag caatgcctac 780

ctataccgca agaggatcca accatgagct cttcccagcc taggggaagc ctggatttgc 840

ccctccatgt aggagctggg cctagggacc tgttgaactc tctcagctga gtcaggggac 900

acctcaggca ctgggacagt tgggcatttg gaggcccgtg tgtgaattcc tgctcctcat 960

gctggagtgc ctcccatttc cttccccttt ctctgtcatc ccagaggaac ataggcatca 1020

tgtgtctgga tgaagctggg gctgcaagac tgcctctcct gcaaggcagc tcatacttgt 1080

actgtatgtt cagaaatttt aggagagaaa aaagtaa 1117

<210> 22

<211> 4396

<212> DNA

<213> Homo sapiens

<220>

<221> transcription factor E2A

<222> (1)..(4396)

<223> GenBank accession No. M31523

<400> 22

gcctgaggtg cccgccctgg ccccaggaga atgaaccagc cgcagaggat ggcgcctgtg 60

ggcacagaca aggagctcag tgacctcctg gacttcagca tgatgttccc gctgcctgtc 120

accaacggga agggccggcc cgcctccctg gccggggcgc agttcggagg ttcaggtctt 180

gaggaccggc ccagctcagg ctcctggggc agcggcgacc agagcagctc ctcctttgac 240

cccagccgga ccttcagcga gggcacccac ttcactgagt cgcacagcag cctctcttca 300

tccacattcc tgggaccggg actcggaggc aagagcggtg agcggggcgc ctatgcctcc 360

ttcgggagag acgcaggcgt gggcggcctg actcaggctg gcttcctgtc aggcgagctg 420

gccctcaaca gccccgggcc cctgtcccct tcgggcatga aggggacctc ccagtactac 480

ccctcctact ccggcagctc ccggcggaga gcggcagacg gcagcctaga cacgcagccc 540

aagaaggtcc ggaaggtccc gccgggtctt ccatcctcgg tgtacccacc cagctcaggt 600

gaggactacg gcagggatgc caccgcctac ccgtccgcca agacccccag cagcacctat 660

cccgccccct tctacgtggc agatggcagc ctgcacccct cagccgagct ctggagtccc 720

ccgggccagg cgggcttcgg gcccatgctg ggtgggggct catccccgct gcccctcccg 780

cccggtagcg gcccggtggg cagcagtgga agcagcagca cgtttggtgg cctgcaccag 840

cacgagcgta tgggctacca gctgcatgga gcagaggtga acggtgggct cccatctgca 900

tcctccttct cctcagcccc cggagccacg tacggcggcg tctccagcca cacgccgcct 960

gtcagcgggg ccgacagcct cctgggctcc cgagggacca cagctggcag ctccggggat 1020

gccctcggca aagcactggc ctcgatctac tccccggatc actcaagcaa taacttctcg 1080

tccagccctt ctacccccgt gggctccccc cagggcctgg caggaacgtc acagtggcct 1140

cgagcaggag cccccggtgc cttatcgccc agctacgacg ggggtctcca cggcctgcag 1200

agtaagatag aagaccacct ggacgaggcc atccacgtgc tccgcagcca cgccgtgggc 1260

acagccggcg acatgcacac gctgctgcct ggccacgggg cgctggcctc aggtttcacc 1320

ggccccatgt cgctgggtgg gcggcacgca ggcctggttg gaggcagcca ccccgaggac 1380

ggcctcgcag gcagcaccag cctcatgcac aaccacgcgg ccctccccag ccagccaggc 1440

accctccctg acctgtctcg gcctcccgac tcctacagtg ggctagggcg agcaggtgcc 1500

acggcggccg ccagcgagat caagcgggag gagaaggagg acgaggagaa cacgtcagcg 1560

gctgaccact cggaggagga gaagaaggag ctgaaggccc cccgggcccg gaccagccca 1620

gacgaggacg aggacgacct tctcccccca gagcagaagg ccgagcggga gaaggagcgc 1680

cgggtggcca ataacgcccg ggagcggctg cgggtccgtg acatcaacga ggcctttaag 1740

gagctggggc gcatgtgcca actgcacctc aacagcgaga agccccagac caaactgctc 1800
atcctgcacc aggctgtctc ggtcatcctg aacttggagc agcaagtgcg agagcggaac 1860
ctgaatccca aagcagcctg tttgaaacgg cgagaagagg aaaaggtgtc aggtgtggtt 1920
ggagaccccc agatggtgct ttcagctccc cacccaggcc tgagcgaagc ccacaacccc 1980
gccgggcaca tgtgaaaggt atgcctccgt gggacgagcc acccgctttc agccctgtgc 2040
tctggcccca gaagccggac tcgagacccc gggcttcatc cacatccaca cctcacacac 2100
ctgttgtcag catcgagcca acaccaacct gacaaggttc ggagtgatgg gggcggccaa 2160
ggtgacactg ggtccaggag ctccctgggg ccctggccta ccactcactg gcctcgctcc 2220
ccctgtcccc gaatctcagc caccgtgtca ctctgtgacc tgtcccatgg atcctgaaac 2280
tgcatcttgg ccctgttgcc tgggctgaca ggagcatttt ttttttttcc agtaaacaaa 2340
acctgaaagc aagcaacaaa acatacactt tgtcagagaa gaaaaaaatg ccttaactat 2400
aaaaagcgga gaaatggaaa catatcactc aagggggatg ctgtggaaac ctggcttatt 2460
cttctaaagc caccagcaaa ttgtgcctaa gcgaaatatt ttttttaagg aaaataaaaa 2520
cattagttac aagatttttt ttttcttaag gtagatgaaa attagcaagg atgctgcctt 2580
tggtctctgg tttttttaag ctttttttgc atatgttttg taagcaacaa atttttttgt 2640
ataaaagtcc cgtgtctctc gctatttctg ctgctgttcc tagactgagc attgcatttc 2700
ttgatcaacc agatgattaa acgttgtatt aaaaagaccc cgtgtaaacc tgagcccccc 2760
ccgtcccccc ccccggaagc cactgcacac agacagacgg ggacaggcgg cgggtctttt 2820
gtttttttga tgttgggggt tctcttggtt ttgtcatgtg gaaagtgatg cgtgggcgtt 2880
ccctgatgaa ggcaccttgg ggcttccctg ccgcatcctc tcccctcagg aaggggactg 2940
acctgggctt gggggaaggg acgtcagcaa ggtggctctg accctcccag gtgactctgc 3000
caagcagctg tggccccagc ggtaccctac acaacgccct ccccaggccc ccctaagctg 3060
ctctcccttg gaacctgcac agctctctga aatggggcat tttgttggga ccagtgaccc 3120
ctggcatggg gaccacaccc tggagcccgg tgctggggac ctcctggaca ccctgtcctt 3180
cactccttgc cccagggacc caggctcatg ctctgaactc tggctgagag gagtctgctc 3240
aggagccagc acaggacacc ccccacccca ccccaccatg tccccattac accagagggc 3300
catcgtgacg tagacaggat gccaggggcc tgaccagcct ccccaatgct ggggagcatc 3360
cctggcctgg ggccacacct gctgccctcc ctctgtgtgg tccaagggca agagtggctg 3420

gagccggggg actgtgctgg tctgagcccc acgaaggcct tgggctgtgg ctccgaccct 3480

gctgcagaac cagcagggtg tcccctcggg cccatctgtg tcccatgtcc cagcacccag 3540

gcctctctcc aggtctcctt ttctggtctt ttgccatgag ggtaaccagc tcttcccagc 3600

tggctgggac tgtcttgggt ttaaaactgc aagtctccta ccctgggatc ccatccagtt 3660

ccacacgaac tagggcagtg gtcactgtgg cacccaggtg tgggcctggc tagctggggg 3720

ccttcatgtg cccttcatgc ccctccctgc attgaggcct tgtggacccc tgggctggct 3780

gtgttcatcc ccgctgcagg tcgggcgtct ccccccgtgc cactcctgag actccaccgt 3840

tacccccagg agatcctgga ctgcctgact cccctcccca gactggcttg ggagcctggg 3900

ccccatggta gatgcaaggg aaacctcaag gccagctcaa tgcctggtat ctgcccccag 3960

tccaggccag gcggagggga ggggctgtcc ggctgcctct cccttctcgg tggcttcccc 4020

tgcgccctgg gagtttgatc tcttaaggga acttgcctct ccctcttgtt ttgctcctgc 4080

cctgccccta ggtctgggtg gcagtggccc catagcctct ggaactgtgc gttctgcata 4140

gaattcaaac gagattcacc cagcgcgagg aggaagaaac agcagttcct gggaaccaca 4200

attatggggg gtggggggtg tgatctgagt gcctcaagat ggttttcaaa aaattttttt 4260

taaagaaaat aattgtatac gtgtcaacac agctggctgg atgattggga ctttaaaacg 4320

accctctttc aggtggattc agagacctgt cctgtatata acagcactgt agcaataaac 4380

gtgacatttt ataaag 4396

<210> 23

<211> 4133

<212> DNA

<213> Homo sapiens

<220>

<221> KIAA0691

<222> (1)..(4133)

<223> GenBank accession No. AB014591

<400> 23

aaattctccc tgggggtaag gtaccagccc tgtcctttat gggcttcttg ttctaaagca 60

tatccgtccc atatggttgc tgctagtcac atgtggtgat tagtaactag ttaaaaatga 120

aaaattcagt tcctccatta cacttgccac atttcagatg ttcagtggcc aacagatatg 180

cgcaaataga gtgtttccag cattgcaaag ttctgttgga tagcactgtt tgccagatgt 240

tcccttcttt gtgggtgagg actcttttgg tgtgacttcc ctctgtattg aggctcttgt 300

tcctcagtat ggggctgttt ctgtctttac agtaagtgac tactccaggg ttccctgccc 360

tgcacacgta gagtgggagc ggcccgtgga tcccagggaa ctgtgctttt cattgtaggc 420

cccctccctg gaggggaaga gggcaatctc cgctggtatc tcagaagtct tcttctgagg 480

cataagcctc tcttcccagg gctcccctgg tctcgctgtc aggccctaag gtatgtcttc 540

ccttggacta aagctccttg gaactccctt ttgacctcag tcttctctgg gttccaggta 600

acttccttta aaataaagac gctcctctct tgaagttttg ggttcctgcc ctgatggtct 660

atgtctccct gactctaaat taccaatcca cttgctatgg gattcctcca tgagtgcaga 720

ttggctccct cacagctgcg gtacctttgc accctcttat cttagtaaga tttctgtctt 780

ctcccaggtc tctcttgggt actgccttct gcccccaaat ctctaagcct tcttggtatt 840

agcttctttg ggttaggagt gttatttcct tttggtttaa ggatcctgct ctggaataaa 900

tgtcttggtg gtttgagtcc cttctacttg gcattcagcc ctgtctgcat gagcgggttc 960

agctcttcac agctttcggc atctctgctc gccgtcgttt tcccccaccc ccaatctttc 1020

ttctcctacc tacagcttac acacacacac acacacacac acacacacac acgcccttct 1080

ctgtgagctg ccagtttcat ttgtctcctg acttgtctga gggatgacct ctcctagcca 1140

cctctgccca gcccctctga gtaggaagtg tgatttccag ggctaatgcc tccatcccag 1200

tcatcagctg tgtgcagcat gactgtcctg ctctgaaaaa cctttttgag tgtattctgg 1260

gggaaggtac tccatgctct aggaattttc cacttcctga gtcagaggca cacaaaaaag 1320

tatgtaactt ttcttgtttc aacaaactta tggggtcccc tgttggccag acactatgct 1380

gggcagtcaa gcgagcatca ggagaactgg ggctggtctc ttgtcagata gcaaatgctt 1440

cttctcttta ccagtcccac ctacctcact atgctgacta ggtccatgtc tctgggtttt 1500

taccagccag ggaatacgtg ttaattcctc tccaatctct cctagcagcg tccgtctcca 1560

agagagtatg aagagagtgc gtctgtaggg cagggaagat ggcggacaag cgcaaactcc 1620

aaggtgagat tgatcgctgc ctcaagaagg tgtccgaggg cgtggagcag tttgaagata 1680

tttggcagaa gctccacaat gcagccaacg cgaaccagaa agaaaagtat gaggctgacc 1740

taaagaagga gattaagaag ctacaacggc tgagggacca aatcaagaca tgggtagcgt 1800

ccaacgagat caaggacaag aggcagctta tagacaaccg caagctcatt gagacgcaaa 1860

tggaacggtt caaagttgtg gaacgagaga ccaaaaccaa agcttacagc aaagagggcc 1920

tgggcctggc ccagaaggta gatcctgccc agaaggagaa ggaagaggtt ggccagtggc 1980

tcacgaatac catcgacacg ctcaacatgc aggtggacca gtttgagagt gaagtggagt 2040

cactgtcagt gcagacacgc aagaagaagg gcgacaagga taagcaggac cggattgagg 2100

gcttgaagcg gcacatcgag aagcaccgct accacgtgcg catgctagag accatcctgc 2160

gcatgctgga caatgactcc atcctcgttg acgccatccg caagatcaag gacgacgttg 2220

agtactatgt tgactcatcc caggaccccg acttcgagga gaacgagttt ctctacgatg 2280

acctggacct cgaggacatt ccacaggcgc tggtcgccac ctcccccccc agccacagcc 2340

acatggagga tgagatcttc aaccagtcca gcagcacgcc cacctcaacc acctccagct 2400

ctcccatccc gcccagccca gccaactgta ccacggaaaa ctctgaagat gataagaaga 2460

ggggacgttc cacagacagt gaagtcagcc agtctccagc caaaaacggc tccaagcctg 2520

tccacagcaa ccagcaccct cagtccccag ctgtgccgcc cacctacccc tccggccccc 2580

cgcctgctgc ctctgccttg agcaccactc ctggcaacaa tggggtcccc gcccccgcag 2640

cacccccaag tgccctgggc cccaaggcca gtccagctcc cagccacaac tcgggcaccc 2700

ctgctcccta tgcccaggct gtggccccac cagctcccag tgggcccagc acgacccagc 2760

cccggccccc cagcgtccag cctagcggag gcggaggcgg cggcagcgga ggcggaggga 2820

gcagcagcag tagtaacagc agtgccggtg gaggggctgg caagcagaat ggcgccacca 2880

gttacagctc agttgtggca gacagcccgg cagaggtggc tttgagcagc agtgggggca 2940

acaatgccag cagccaggcc ttgggccccc cttccggccc ccacaaccca cctcccagca 3000

cctcgaagga acccagtgcg gcagccccaa cgggggctgg gggcgtggcc ccaggctcag 3060

ggaacaactc agggggaccc agcctcctgg tgccactgcc tgtgaatcct cccagctccc 3120

caacgcccag cttcagtgat gccaaggcag ccggtgccct gctcaatggg cctccacagt 3180

tcagcaccgc cccagaaatc aaggcccctg agcctctgag ctccttgaag tccatggcgg 3240

aacgggcagc catcagctct ggcattgagg accctgtgcc aacgctgcac ctgaccgagc 3300

gagacatcat cctgagcagt acatcagcac ctccggcctc agcccagccg cccctgcagc 3360

tgtcagaggt gaacataccg ctgtcgctgg gtgtctgtcc actgggccct gtgcccctca 3420

ccaaggagca gctctatcag caggccatgg aagaggccgc ctggcaccac atgcctcacc 3480

cctctgactc tgagcgtatt cggcagtacc tcccccggaa cccctgtccg acgcccccct 3540

accaccacca gatgccaccc ccacactcgg acactgtgga attctaccag cgcctgtcga 3600

ccgagacact cttcttcatc ttctactatc tggagggcac taaggcacag tatctggcag 3660

ccaaggccct aaagaagcag tcatggcgat tccacaccaa gtacatgatg tggttccaga 3720

ggcacgagga gcccaagacc atcactgacg agtttgagca gggcacctac atctactttg 3780

actacgagaa gtggggccag cggaagaagg aaggcttcac ctttgagtac cgctacctgg 3840

aggaccggga cctccagtga caccggcccc tccctctacc cacccccttc ccccgcatgc 3900

tgatccccct gcccaggtga gggccctgcc ctggaagact ggagggaggc cccaagccac 3960

ggggcatccc cctctcccag gaagcaggga gggggccggg aggttttcct ctcagcccca 4020

ccctgggggc ccgggggcga gggctgcccc ctcctcccct ccccagtgag ggacattttt 4080

tggtaaacct attttcattt tggaaaatat ttatgaataa atagttttat atg 4133

<210> 24

<211> 1260

<212> DNA

<213> Homo sapiens

<220>

<221> serotonin 1D receptor 5-HT1D

<222> (1)..(1260)

<223> Genank accession No. M81590

<400> 24

ccctctcctt cgtccgctcc atgcccaaga gctgcgctcc ggagctgggg cgaggagagc 60

catggaggaa ccgggtgctc agtgcgctcc accgccgccc gcgggctccg agacctgggt 120

tcctcaagcc aacttatcct ctgctccctc ccaaaactgc agcgccaagg actacattta 180

ccaggactcc atctccctac cctggaaagt actgctggtt atgctattgg cgctcatcac 240

cttggccacc acgctctcca atgcctttgt gattgccaca gtgtaccgga cccggaaact 300

gcacaccccg gctaactacc tgatcgcctc tctggcagtc accgacctgc ttgtgtccat 360

cctggtgatg cccatcagca ccatgtacac tgtcaccggc cgctggacac tgggccaggt 420

ggtctgtgac ttctggctgt cgtcggacat cacttgttgc actgcctcca tcctgcacct 480

ctgtgtcatc gccctggacc gctactgggc catcacggac gccgtggagt actcagctaa 540

aaggactccc aagagggcgg cggtcatgat cgcgctggtg tgggtcttct ccatctctat 600

ctcgctgccg cccttcttct ggcgtcaggc taaggccgaa gaggaggtgt cggaatgcgt 660

ggtgaacacc gaccacatcc tctacacggt ctactccacg gtgggtgctt tctacttccc 720

caccctgctc ctcatcgccc tctatggccg catctacgta gaagcccgct cccggatttt 780

gaaacagacg cccaacagga ccggcaagcg cttgacccga gcccagctga taaccgactc 840

ccccgggtcc acgtcctcgg tcacctctat taactcgcgg gttcccgacg tgcccagcga 900

atccggatct cctgtgtatg tgaaccaagt caaagtgcga gtctccgacg ccctgctgga 960

aaagaagaaa ctcatggccg ctagggagcg caaagccacc aagaccctag ggatcatttt 1020

gggagccttt attgtgtgtt ggctaccctt cttcatcatc tccctagtga tgcctatctg 1080

caaagatgcc tgctggttcc acctagccat ctttgacttc ttcacatggc tgggctatct 1140

caactccctc atcaacccca taatctatac catgtccaat gaggacttta aacaagcatt 1200

ccataaactg atacgtttta agtgcacaag ttgacttgcc gtttgcagtg ggtcgcccta 1260

<210> 25

<211> 1990

<212> DNA

<213> Homo sapiens

<220>

<221> coagulation factor XII (Hageman factor)

<222> (1)..(1990)

<223> GenBank accession No. M31315

<400> 25

ctgctgctcc tggggttcct gctggtgagc ttggagtcaa cactttcgat tccaccttgg 60

gaagccccca aggagcataa gtacaaagct gaagagcaca cagtcgttct cactgtcacc 120

ggggagccct gccacttccc cttccagtac caccggcagc tgtaccacaa atgtacccac 180

aagggccggc caggccctca gccctggtgt gctaccaccc ccaactttga tcaggaccag 240

cgatggggat actgtttgga gcccaagaaa gtgaaagacc actgcagcaa acacagcccc 300

tgccagaaag gagggacctg tgtgaacatg ccaagcggcc cccactgtct ctgtccacaa 360

cacctcactg gaaaccactg ccagaaagag aagtgctttg agcctcagct tctccggttt 420

ttccacaaga atgagatatg gtatagaact gagcaagcag ctgtggccag atgccagtgc 480

aagggtcctg atgcccactg ccagcggctg gccagccagg cctgccgcac caacccgtgc 540

ctccatgggg gtcgctgcct agaggtggag ggccaccgcc tgtgccactg cccggtgggc 600

tacaccggac ccttctgcga cgtggacacc aaggcaagct gctatgatgg ccgcgggctc 660

agctaccgcg gcctggccag gaccacgctc tcgggtgcgc cctgtcagcc gtgggcctcg 720

gaggccacct accggaacgt gactgccgag caagcgcgga actggggact gggcggccac 780

gccttctgcc ggaacccgga caacgacatc cgcccgtggt gcttcgtgct gaaccgcgac 840

cggctgagct gggagtactg cgacctggca cagtgccaga ccccaaccca ggcggcgcct 900

ccgaccccgg tgtcccctag gcttcatgtc ccactcatgc ccgcgcagcc ggcaccgccg 960

aagcctcagc ccacgacccg gaccccgcct cagtcccaga ccccgggagc cttgccggcg 1020

aagcgggagc agccgccttc cctgaccagg aacggcccac tgagctgcgg gcagcggctc 1080

cgcaagagtc tgtcttcgat gacccgcgtc gttggcgggc tggtggcgct acgcggggcg 1140

cacccctaca tcgccgcgct gtactggggc cacagtttct gcgccggcag cctcatcgcc 1200

ccctgctggg tgctgacggc cgctcactgc ctgcaggacc ggcccgcacc cgaggatctg 1260

acggtggtgc tcggccagga acgccgtaac cacagctgtg agccgtgcca gacgttggcc 1320

gtgcgctcct accgcttgca cgaggccttc tcgcccgtca gctaccagca cgacctggct 1380

ctgttgcgcc ttcaggagga tgcggacggc agctgcgcgc tcctgtcgcc ttacgttcag 1440

ccggtgtgcc tgccaagcgg cgccgcgcga ccctccgaga ccacgctctg ccaggtggcc 1500

ggctggggcc accagttcga gggggcggag gaatatgcca gcttcctgca ggaggcgcag 1560

gtaccgttcc tctccctgga gcgctgctca gccccggacg tgcacggatc ctccatcctc 1620

cccggcatgc tctgcgcagg gttcctcgag ggcggcaccg atgcgtgcca gggtgattcc 1680

ggaggcccgc tggtgtgtga ggaccaagct gcagagcgcc ggctcaccct gcaaggcatc 1740

atcagctggg gatcgggctg tggtgaccgc aacaagccag gcgtctacac cgatgtggcc 1800

tactacctgg cctggatccg ggagcacacc gtttcctgat tgctcaggga ctcatctttc 1860

cctccttggt gattccgcag tgagagagtg gctggggcat ggaaggcaag attgtgtccc 1920

attcccccag tgcggccagc tccgcgccag gatggcgagg aactcaataa agtgctttga 1980

aaatgctgag 1990

<210> 26

<211> 3840

<212> DNA

<213> Homo sapiens

<220>

<221> proto-oncogene tyrosine-protein kinase c-abl

<222> (1)..(3840)

<223> GenBank accession No. M14752

<400> 26

ggccttcccc ctgcgaggat cgccgttggc ccgggttggc tttggaaagc ggcggtggct 60

ttgggccggg ctcggcctcg ggaacgccag gggcccctgg gtgcggacgg gcgcggccag 120

gagggggtta aggcgcaggc ggcggcgggg cgggggcggg cctggcgggc gccctctccg 180

ggccctttgt taacaggcgc gtcccggcca gcggagacgc ggccgccctg ggcgggcgcg 240

ggcggcgggc ggcggtgagg gcggcctgcg ggcggcgccc cgggggccgg gccgagccgg 300

gcctgagccg ggcccggacc gagctgggag aggggctccg gcccgatcgt tcgcttggcg 360

caaaatgttg gagatctgcc tgaagctggt gggctgcaaa tccaagaagg ggctgtcctc 420

gtcctccagc tgttatctgg aagaagccct tcagcggcca gtagcatctg actttgagcc 480

tcagggtctg agtgaagccg ctcgttggaa ctccaaggaa aaccttctcg ctggacccag 540

tgaaaatgac cccaaccttt tcgttgcact gtatgatttt gtggccagtg gagataacac 600

tctaagcata actaaaggtg aaaagctccg ggtcttaggc tataatcaca atggggaatg 660

gtgtgaagcc caaaccaaaa atggccaagg ctgggtccca agcaactaca tcacgccagt 720

caacagtctg gagaaacact cctggtacca tgggcctgtg tcccgcaatg ccgctgagta 780

tccgctgagc agcgggatca atggcagctt cttggtgcgt gagagtgaga gcagtcctag 840

ccagaggtcc atctcgctga gatacgaagg gagggtgtac cattacagga tcaacactgc 900

ttctgatggc aagctctacg tctcctccga gagccgcttc aacaccctgg ccgagttggt 960

tcatcatcat tcaacggtgg ccgacgggct catcaccacg ctccattatc cagccccaaa 1020

gcgcaacaag cccactgtct atggtgtgtc ccccaactac gacaagtggg agatggaacg 1080

cacggacatc accatgaagc acaagctggg cgggggccag tacggggagg tgtacgaggg 1140

cgtgtggaag aaatacagcc tgacggtggc cgtgaagacc ttgaaggagg acaccatgga 1200

ggtggaagag ttcttgaaag aagctgcagt catgaaagag atcaaacacc ctaacctagt 1260

gcagctcctt ggggtctgca cccgggagcc cccgttctat atcatcactg agttcatgac 1320

ctacgggaac ctcctggact acctgaggga gtgcaaccgg caggaggtga acgccgtggt 1380

gctgctgtac atggccactc agatctcgtc agccatggag tacctagaga agaaaaactt 1440

catccacaga gatcttgctg cccgaaactg cctggtaggg gagaaccact tggtgaaggt 1500

agctgatttt ggcctgagca ggttgatgac aggggacacc tacacagccc atgctggagc 1560

caagttcccc atcaaatgga ctgcacccga gagcctggcc tacaacaagt tctccatcaa 1620

gtccgacgtc tgggcatttg gagtattgct ttgggaaatt gctacctatg gcatgtcccc 1680

ttacccggga attgaccgtt cccaggtgta tgagctgcta gagaaggact accgcatgaa 1740

gcgcccagaa ggctgcccag agaaggtcta tgaactcatg cgagcatgtt ggcagtggaa 1800

tccctctgac cggccctcct ttgctgaaat ccaccaagcc tttgaaacaa tgttccagga 1860

atccagtatc tcagacgaag tggaaaagga gctggggaaa caaggcgtcc gtggggctgt 1920

gactaccttg ctgcaggccc cagagctgcc caccaagacg aggacctcca ggagagctgc 1980

agagcacaga gacaccactg acgtgcctga gatgcctcac tccaagggcc agggagagag 2040

cgatcctctg gaccatgagc ctgccgtgtc tccattgctc cctcgaaaag agcgaggtcc 2100

cccggagggc ggcctgaatg aagatgagcg ccttctcccc aaagacaaaa agaccaactt 2160

gttcagcgcc ttgatcaaga agaagaagaa gacagcccca acccctccca aacgcagcag 2220

ctccttccgg gagatggacg gccagccgga gcgcagaggg gccggcgagg aagagggccg 2280

agacatcagc aacggggcac tggctttcac ccccttggac acagctgacc cagccaagtc 2340

cccaaagccc agcaatgggg ctggggtccc caatggagcc ctccgggagt ccgggggctc 2400

aggcttccgg tctccccacc tgtggaagaa gtccagcacg ctgaccagca gccgcctagc 2460

caccggcgag gaggagggcg gtggcagctc cagcaagcgc ttcctgcgct cttgctccgt 2520

ctcctgcgtt ccccatgggg ccaaggacac ggagtggagg tcagtcacgc tgcctcggga 2580

cttgcagtcc acgggaagac agtttgactc gtccacattt ggagggcaca aaagtgagaa 2640

gccggctctg cctcggaaga gggcagggga gaacaggtct gaccaggtga cccgaggcac 2700

agtaacgcct ccccccaggc tggtgaaaaa gaatgaggaa gctgctgatg aggtcttcaa 2760

agacatcatg gagtccagcc cgggctccag cccgcccaac ctgactccaa aacccctccg 2820

gcggcaggtc accgtggccc ctgcctcggg cctcccccac aaggaagaag cctggaaagg 2880

cagtgcctta gggacccctg ctgcagctga gccagtgacc cccaccagca aagcaggctc 2940

aggtgcacca aggggcacca gcaagggccc cgccgaggag tccagagtga ggaggcacaa 3000

gcactcctct gagtcgccag ggagggacaa ggggaaattg tccaagctca aacctgcccc 3060

gccgccccca ccagcagcct ctgcagggaa ggctggagga aagccctcgc agaggcccgg 3120

ccaggaggct gccggggagg cagtcttggg cgcaaagaca aaagccacga gtctggttga 3180

tgctgtgaac agtgacgctg ccaagcccag ccagccggca gagggcctca aaaagcccgt 3240

gctcccggcc actccaaagc cacaccccgc caagccgtcg gggaccccca tcagcccagc 3300

ccccgttccc ctttccacgt tgccatcagc atcctcggcc ttggcagggg accagccgtc 3360

ttccactgcc ttcatccctc tcatatcaac ccgagtgtct cttcggaaaa cccgccagcc 3420

tccagagcgg gccagcggcg ccatcaccaa gggcgtggtc ttggacagca ccgaggcgct 3480

gtgcctcgcc atctctggga actccgagca gatggccagc cacagcgcag tgctggaggc 3540

cggcaaaaac ctctacacgt tctgcgtgag ctatgtggat tccatccagc aaatgaggaa 3600

caagtttgcc ttccgagagg ccatcaacaa actggagaat aatctccggg agcttcagat 3660

ctgcccggcg tcagcaggca gtggtccggc ggccactcag gacttcagca agctcctcag 3720

ttcggtgaag gaaatcagtg acatagtgca gaggtagcag cagtcagggg tcaggtgtca 3780

ggcccgtcgg agctgcctgc agcacatgcg ggctcgccca tacccatgac agtggctgag 3840

<210> 27

<211> 1628

<212> DNA

<213> Homo sapiens

<220>

<221> AF1q

<222> (1)..(1628)

<223> GenBank accession No. U16954

<220>

<221> misc_feature

<222> (114)..(114)

<223> undefined nucleotide

<400> 27

agtcagcacg ggggtgctgg aagagatcgg gaataatagc gcagaccaat gagcctaggg 60

agatgctttc atcgtctctc cttccctcaa gtgttctgga acctatcatt tganttagcc 120

gagtcaggca ggagggggcg gggaatcctt ccgcccttct taggaggggc tgcattgcag 180

ggggagagtg aactgacaga ctcagtcact gaagagggaa aaggagtgag aagacaaagc 240

cgtcaaagcc ccaacagctt tgtatttctc cagcccggcc ggcagacccc ggagctcccg 300

aggcactccc tccatctttg gaacgcgcca gtaattgaat tgataacagg aagctatgag 360

ggaccctgtg agtagccagt acagttcctt tcttttctgg aggatgccca tcccagaact 420

ggatctgtcg gagctggaag gcctgggtct gtcagataca gccacctaca aggtcaaaga 480

cagcagcgtt ggcaaaatga tcgggcaagc aactgcagca gaccaggaga aaaaccctga 540

aggtgatggc ctccttgagt acagcacctt caacttctgg agagctccca ttgccagcat 600

ccactccttc gaactggact tgctctaagg ccaagacttc tctctcccat caccttgccc 660

tcattgtctt ccctctcaag ccccttcctt tccactcctt tcccatttta atcttgttct 720

ctccctactg tgttggtggt gctgatgaat ctgccagagt tgagttctat gtatttattt 780

atctatctgt ctactccatt tctctcaaaa gccctcaagt cacaaagtaa atggttcaag 840

caatggagta ctgggtcaca gggattcctc ctttcccccc caaatattaa ctccagaaac 900

taggcctgac tggggacacc ctgagagtag tatagtagtg caaaatggaa gactgatttt 960

tgactctatt ataatcagct tcagagattc cttaaacctt cctaatttcc tgctccaggg 1020

cagtgaaaca caaatatttc ttcaaggggt gatgaaaacc tcggaagttt taatttgagg 1080

ttatctgcta cgaaacagta tttctaaaag gctaaagtga taagtctctt gctttttttt 1140

gatcctgctc ttatattctt ttttttcctc agagaaatca ggagggtagt tagaggtata 1200

aaacaggagg aaatattatg gaaaatgaaa atagggaaaa taattgaatc attttagaag 1260

tagctaattt cttttctcaa aagagtgtcc cttcttcaca cctactcact ttacaacttt 1320

gctcctaact gtgggttgaa aactctagct aaagaaagtt atcaaatctt aacatgcatt 1380

cctactatta tgatagtttt taaggtttca attcaatctt ctgaacggca taagtcctat 1440

tttagcctta cctcctgcat ttgcaatacg taatactgat cagtgggcac agttcttcag 1500

ctacattgag accctgaaat gaacaattat attctgactc gacatcttgt ccccaatcct 1560

tccaaaaata ttgatggtga tttgtgctac catttactcg tttatttaat aaagacattc 1620

aattccca 1628

<210> 28

<211> 993

<212> DNA

<213> Homo sapiens

<220>

<221> guanidinoacetate N-methyltransferase

<222> (1)..(993)

<223> GenBank accession No. Z49878

<400>  28

cggcggcgcg cgatcgaggt cgggtcgccg tccagcctgc agcatgagcg cccccagcgc      60

gacccccatc ttcgcgcccg gcgagaactg cagccccgcg tggggggcgg cgcccgcggc      120

ctacgacgca gcggacacgc acctgcgcat cctgggcaag ccggtgatgg agcgctggga      180

gaccccctat atgcacgcgc tggccgccgc cgcctcctcc aaagggggcc gggtcctgga      240

ggtgggcttt ggcatggcca tcgcagcgtc aaaggtgcag gaggcgccca ttgatgagca      300

ttggatcatc gagtgcaatg acggcgtctt ccagcggctc cgggactggg ccccacggca      360

gacacacaag gtcatcccct tgaaaggcct gtgggaggat gtggcaccca ccctgcctga      420

cggtcacttt gatgggatcc tgtacgacac gtacccactc tcggaggaga cctggcacac      480

acaccagttc aacttcatca agaaccacgc ctttcgcctg ctgaagccgg ggggcgtcct      540

cacctactgc aacctcacct cctgggggga gctgatgaag tccaagtact cagacatcac      600

catcatgttt gaggagacgc aggtgcccgc gctgctggag gccggcttcc ggagggagaa      660

catccgtacg gaggtgatgg cgctggtccc accggccgac tgccgctact acgccttccc      720

acagatgatc acgcccctgg tgaccaaagg ctgagccccc accccggccc ggccacaccc      780

atgccctccg ccgtgccttc ctggccggga gtccagggtg tcgcaccagc cctgggctga      840

tcccagctgt gtgtcaccag aagctttccc ggcttctctg tgaggggtcc caccagccca      900

gggctgatcc cagctgtgtg tcaccagcag ctttcccagc ttgctctgtg agggtcactg      960

ctgcccactg cagggtgccc tgaggtgaag ccg                                  993

<210>  29

<211>  835

<212>  DNA

<213>  Homo sapiens

<220>

<221>  interferon-induced nuclear phosphoprotein

<222>  (1)..(835)

<223>  Genbank accession No. as of 09 Dec 2002: L22342

<400>  29

atggcgagca gcggagtcaa gaacacacca cgatggcgga gaaaagcccc tcatgggagg     60

gaaaggaaag agaaaggaaa gaaaagaaaa agatgtatct ggtcaactcc aaaaaggaga    120

cataagaaaa aaagcctccc aagagagatc attgatggca cttcagaaat gaatgaagga    180

aagaggtccc agaagatgcc tagtacacca cgaagggtca cacaaggggc agcctcacct    240

gggcatggca tccaagagaa gctccaagtg gtggataagg tgactcaaag gaaagacgac    300

tcaacctgga actcagaggt catgatgagg gtccaaaagg caagaactaa atgtgcccga    360

aagtccagat cgaaagaaaa gaaaaaggag aaagatatct gttcaagctc aaaaaggaga    420

tttcagaaaa atattcaccg aagaggaaaa cccaaaagtg acactgtgga ttttcactgt    480

tctaagtccc ccgtgacctg tggtgaggcg aaagggattt tatataagaa gaaaatgaaa    540

cacggatcct cagtgaagtg cattcggaat gaggatggaa cttggttaac accaaatgaa    600

tttgaagtcg aaggaaaagg aaggaacgca aagaactgga aacggaatat acgttgtgaa    660

ggaatgaccc taggagagct gctgaagagt ggacttttgc tctgtcctcc aagaataaat    720

ctcaagagag agttaaatag caagtgaatt tctactaccc tctcagtcac catgttgcag    780

actttccctg tctggaggct caccttagag cttctgagtt tccaagcccg gaatt         835